# EUROPEAN PATENT SPECIFICATION

(11) **EP 1 280 795 B1**
(45) Date of publication and mention of the grant of the patent: **23.03.2005**
(21) Application number: 00992946.4
(22) Date of filing: 25.10.2000
(51) Int. Cl.: C07D 403/00

(54) **N-SUBSTITUTED CARBAMOYLOXYALKYL-AZOLIUM DERIVATIVES**
N-CARBAMOYLOXYALKYL-SUBSTITUIERTE AZOLIUM-VERBINDUNGEN
DERIVES CARBAMOYLOXYALKYL-AZOLIUM N-SUBSTITUES

(30) Priority: 02.11.1999 EP 99121694
(43) Date of publication of application: 05.02.2003
(73) Proprietor: Basilea Pharmaceutica AG, 4005 Basel (CH)
(72) Inventor: Fukuda, Hiroshi, Ohta-ku, Tokyo (JP); Hayase, Tadakatsu Nippon Roche Shonan Dor. R.A-103, Chigasaki-shi, Kanagawa-ken 253-0054 (JP); Mizuguchi, Eisaku, Kamakura-shi, Kanagawa-ken 247-0074 (JP); Shimma, Nobuo, Chigasaki-shi, Kanagawa-ken 253-0054 (JP); Ohwada, Jun, Kamakura-shi, Kanagawa-ken 247-0074 (JP); Oikawa, Nobuhiro, Carlton Pla. Kaw. R. 802, Kawasaki-shi, Kanagawa-ken 210-0024 (JP); Sakaitani, Masahiro, Puranbehru Shonan Chigasaki, Chigasaki-shi, Kanagawa-ken 253-0012 (JP); Tsukazaki, Masao, Fujisawa-shi, Kanagawa-ken 252-0805 (JP); Umeda, Isao, Imp. Higashi Hakuraku Gardenhouse (B), Yokohama-shi, Kanagawa-ken 221-0073 (JP)
(74) Representative: Zimmermann, Hans, Dr.
(86) International application number: PCT/EP2000/010524
(87) International publication number: WO 2001/032652

(56) References cited:
- WO-A-98/43970
- WO-A-99/45008
- PATENT ABSTRACTS OF JAPAN vol. 1999, no. 13, 30 November 1999 (1999-11-30) & JP 11 228548 A (TAKEDA CHEM IND LTD), 24 August 1999 (1999-08-24)
- DAVIDSEN, S. K. ET AL.: "N-(Acyloxyalkyl)pyridinium salts as soluble prodrugs of a potent platelet activating factor antagonist" J. MED. CHEM., vol. 37, no. 26, 1994, pages 4423-4429, XP002166849
- SUNDBERG, R. J. ET AL.: "Synthesis of omega-carbamoyloxyalkylimidazolium salts for evaluation as protective agents against acetylcholinesterase intoxication by soman" MED. CHEM. RES. , vol. 7, no. 2, 1997, pages 123-136, XP000996486
- PATONAY, T. ET AL.: "alfa-Haloalkyl haloformates and related compounds 3." SYNTHETIC COMMUNICATIONS, vol. 26, no. 22, 1996, pages 4253-4265, XP000996537

## Description

Although several azole compounds are currently used for systemic mycoses, none of them fulfills the necessary clinical requirement in full extent, i.e. efficacy against major systemic mycoses including disseminated aspergillosis, safety, and oral or parenteral formulations. Particularly, demand of a parenteral administration of the azole compounds is increasing for the treatment of serious systemic mycoses. Most of the azole compounds on the market as well as under development are highly lipophilic molecules that make the parenteral formulation difficult.

Examples for known azole compounds or prodrugs thereof are given in the following references.

WO-A-99 45008 discloses azole derivatives that can be used for the production of medicaments for treating fungal infections and mycoses.

Patent Abstracts of Japan, Vol. 1999, No. 13, 30.11.1999; & JP-A-11 228548 as well as WO-A-98 43970 relate to quaternized nitrogen-containing imidazol-1-yl or 1,2,4-triazol-1-yl compounds that can be converted into antifungal azole compounds.

Davidsen, S.K. et al., J. Med. Chem. 1994, 36(26), 4423-4429 describe N-(acyloxyalkyl)-pyridinium salts as soluble prodrugs of a potent platelet activating factor antagonist.

The present invention relates to novel water soluble azole compounds useful for the treatment of systemic mycoses and suitable for both oral and particularly parenteral administration, a process for their manufacture, antifungal compositions containing them and a method for treating mycoses.

More particularly, the present invention refers to compounds of formula (III), 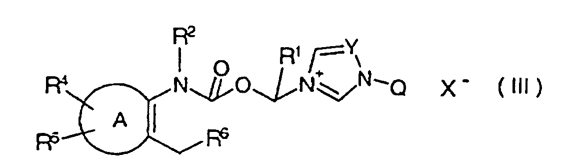 as defined in claim 1.

In this specification the term "alkyl" refers to a branched or unbranched saturated hydrocarbon radical, consisting solely of carbon and hydrogen atoms, having 1 to 6, preferably 1 to 4 carbon atom(s), unless otherwise indicated, e.g. methyl, ethyl, n-propyl, iso-propyl, butyl, iso-butyl or tert-butyl n-pentyl or pentan-3-yl and the like.

The term "5- or 6-membered cycloalkyl" means a monovalent saturated carbocyclic radical, e.g. cyclopentyl and cylcohexyl. The term "optionally substituted 5- or 6-membered cycloalkyl" in the group R³ means a 5- or 6-membered cycloalkyl as defined above optionally substituted with hydroxy, amino, alkylamino, acyloxy, acylamino or acylalkylamino wherein acyl means an easily hydrolyzable radical under physiological condition.

The terms "solvates" and "hydrates" refer to compounds which additionally comprise solvent molecules or, in the case of hydrates, water molecules.

The "optionally substituted phenyl" means a phenyl optionally substituted with aminoalkylcarbonyl, nitro, alkylaminoalkyl, trifluoromethoxy, alkyl, halogen, alkoxy, cyano, or alkylaminoalkylcarbonyloxyalkyl. Other substituents are R⁴, R⁵ and -CH₂-R⁶ as defined above.

The term "optionally substituted pyridin-2-yl" means a pyridin-2-yl optionally substituted with alkylaminoalkylcarbonyloxyalkyl, alkylcarbonyloxyalkyl, or aminoalkylcarbonyloxyalkyl. Other substituents are R⁴, R⁵ and -CH₂-R⁶ as defined above.

Preferably, the terms "optionally substituted phenyl" and "optionally substituted pyridin-2-yl" refer to the group of formula (VI) 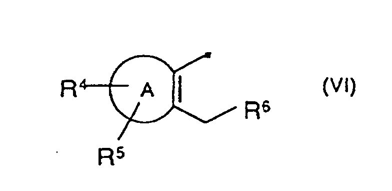 wherein R⁴, R⁵ and R⁶ are as defined above.

Preferably, R⁶ is hydroxy, amino, alkylamino, acyloxy, acylamino or acyl, alkylamino, alkylaminoalkylcarbonyloxy, aminoalkylcarbonyloxy in which acyl means an easily hydrolyzable radical under physiological conditions.

The term "acyl" refers to an easily hydrolyzable radical under physiological conditions which preferably means an acyl residue of an amino acid or a group represented by the formula, R⁷CO- or (R⁸O)₂PO-, wherein R⁷ is hydrogen, alkoxy, alkyl which may be optionally substituted with carboxy, amino, alkylamino, dialkylamino, or aryl, preferably phenyl; and R⁸ is hydrogen or alkyl.). More preferably, "acyl" is formyl, acetyl, propionyl, isobutyryl, pivaloyl, succinoyl, benzoyl, nicotinoyl, phosphoryl, dimethylphosphoryl, aminoacetyl, 3-aminopropionyl, 4-aminobutyryl, (2-amino-acetylamino)-acetyl, (S)-2,5-diaminopentoyl, (S)-2-aminopropionyl, (S)-pyrrolidine-2-carbonyl, (methylamino)acetyl, (propylamino)acetyl, (S)-2-(methylamino)propionyl, 3-(methylamino)propionyl, (S)-2-amino-3-methylbutanoyl, (isopropylamino)acetyl, (2S)-2-(ethylamino)propionyl, (ethylarnino)acetyl and the like.

The term "alkoxy" refers to preferably straight or branched alkyl-O- chain having 1 to 5 carbon atom(s) such as methoxy, ethoxy, n-propoxy, iso-propoxy, n-butoxy, iso-butoxy.

The term "halogen" denotes fluorine, chlorine or bromine.

The term "alkylthio" refers to preferably straight or branched alkyl-S- chain having 1 to 4 carbon atom(s) such as methylthio, ethylthio, n-propylthio.

X⁻ is an anion from a pharmaceutically acceptable inorganic acid, e.g. a mineral acid; such as chloride, bromide or sulfate; or from an organic acid, e.g. an aliphatic, aromatic or arylaliphatic carboxylic or sulfonic acid such as acetoxy, trifluoroacetoxy, mesyloxy anion and the like.

The term "leaving group" refers to chloro, bromo, iodo, tosyloxy, mesyloxy and the like.

The term "carbonyl" refers to the group-C(O)-.

The term "oxy" refers to the group -O-.

The term "amino" refers to -NH₂.

The term "sulfinyl" refers to the group -SO-.

The term "sulfonyl" refers to the group -SO₂-.

The term "sulfo" refers to the group HO-SO₂-.

In a more preferred embodiment, the above compounds may be characterized by formula (III), 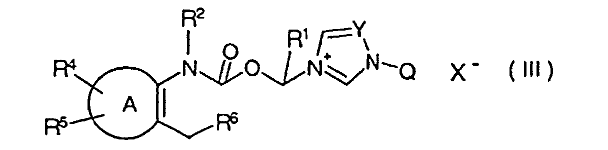 wherein
R¹, R², Q, Y and X are as defined above and
R⁴ and R⁵ are independently selected from the group consisting of hydrogen, halogen, alkyl, alkoxy, alkylthio, alkylsulfinyl, alkylsulfonyl, carboxy, alkoxycarbonyl, cyano, trifluoromethyl, trifluormethoxy, nitro, aminosulfonyl or sulfo;
R⁶ is hydroxy, alkoxycarbonylalkylamino, alkoxycarbonylamino, amino, alkylamino, alkylcarbonyloxy, alkoxycarbonylalkylamino-alkylcarbonyloxy, alkoxycarbonylamino-alkylcarbonyloxy, alkylaminoalkylcarbonyloxy, aminoalkylcarbonyloxy, alkylcarbonylamino, alkylcarbonylalkylamino, acyloxy, acylamino, acylalkylamino;
the group 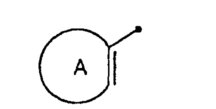 is phenyl or pyridin-2-yl.

In the compounds of the present invention 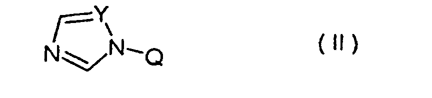 is a group of the following structure:

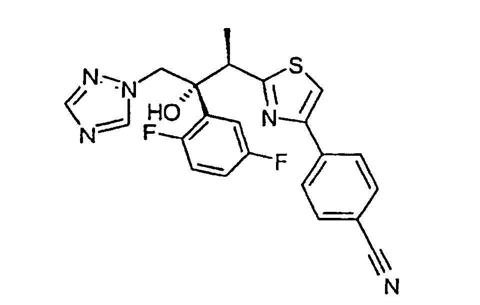

In a further preferred embodiment of the invention are compounds wherein R¹ is hydrogen or alkyl, preferably methyl. R² is hydrogen, alkyl, alkylcarbonyloxyalkyl, alkoxycarbonyl, alkylcarbonyl, mono- or dialkylaminoalkylcarbonyloxyalkyl, preferably hydrogen or alkyl, more preferably alkyl, e.g. methyl.

In the preferred compounds, X is halogen, preferably chlor. In a preferred embodiment of the above invention R⁴ and R⁵ are independently selected from the group consisting of hydrogen, halogen, alkyl, alkoxy, alkylthio, alkylsulfinyl, alkylsulfonyl, carboxy, alkoxycarbonyl, cyano, trifluormethoxy, nitro, aminosulfonyl or sulfo, preferably from hydrogen, halogen, alkoxy, cyano, trifluoromethyl, trifluormethoxy and nitro. In a preferred embodiment, R⁴ and R⁵ independently are selected from hydrogen, halogen and alkoxy, most preferably R⁴ and R⁵ both are hydrogen.

In another preferred embodiment the invention comprises compounds wherein R⁶ is hydroxy, alkoxycarbonylalkylamino, alkoxycarbonylamino, amino, alkylamino, alkylcarbonyloxy, alkoxycarbonylalkylamino-alkylcarbonyloxy, alkoxycarbonylamino-alkylcarbonyloxy, alkylaminoalkylcarbonyloxy, aminoalkylcarbonyloxy, alkylcarbonylamino, alkylcarbonylalkylamino, acyloxy, acylamino, acylalkylamino, acylalkylamino, preferably R⁶ is alkylamino, alkylcarbonyloxy, alkylaminoalkylcarbonyloxy, or aminoalkylcarbonyloxy, more preferably R⁶ is acyloxy in which acyl is the aryl residue of an amino acid such as sarcosyl, alanyl, seryl, cysteinyl and the like, e.g. alkylaminoalkylcarbonyloxy.

In addition the present invention comprises compounds wherein the group 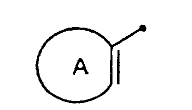 is optionally substituted phenyl or pyridin-2-yl, preferably pyridin-2-yl.

The invention refers to the above compounds wherein Q is

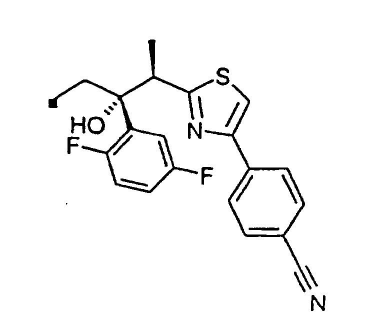

Y is nitrogen, R¹ is alkyl, R² is alkyl, R³ is optionally substituted pyridin-2-yl, X- is halogen, R⁴ and R⁵ are hydrogen, R⁶ is alkylaminoalkylcarbonyloxy, and pharmaceutically acceptable salts, hydrates or solvates thereof.

In particular, the invention refers to the compounds selected from the group consisting of
a) [[N-methyl-N-2-(acetoxymethyl)phenyl]carbamoyloxy]methyl-1-[(2R,3R)-2-(2,5-difluorophenyl)-2-hydroxy-3-[4-(4-cyanophenyl)thiazol-2-yl]butyl]-1H-[1,2,4]triazol-4-ium chloride,
b) 1-[[N-methyl-N-2-(isopropylaminomethyl)phenyl]carbamoyloxy]ethyl-1-[(2R,3R)-2-(2,5-difluorophenyl)-2-hydroxy-3-[4-(4-cyanophenyl)thiazol-2-yl]butyl]-1H-[1,2,4]triazol-4-ium chloride hydrochloride,
c) 1-[[N-methyl-N-3-[(methylamino)acetoxymethyl]pyridin-2-yl]carbamoyloxy]ethyl-1-[(2R,3R)-2-(2,5-difluorophenyl)-2-hydroxy-3-[4-(4-cyanophenyl)thiazol-2-yl]butyl]-1H-[1,2,4]triazol-4-ium chloride dihydrochloride,
d) 1-[[N-methyl-N-3-[(methylamino)acetoxymethyl]pyridin-2-yl]carbamoyloxy]ethyl-1-[(2R,3R)-2-(2,5-difluorophenyl)-2-hydroxy-3-[4-(4-cyanophenyl)thiazol-2-yl]butyl]-1H-[1,2,4]triazol-4-ium chloride hydrochloride,
f) [[N-methyl-N-phenyl]carbamoyloxy]methyl-1-[(2R,3R)-2-(2,5-difluorophenyl)-2-hydroxy-3-[4-(4-cyanophenyl)thiazol-2-yl]butyl]-1H-[1,2,4jtriazol-4-ium chloride,
g) 1-[[N-methyl-N-3-(acetoxymethyl)pyridin-2-yl]carbamoyloxy]ethyl-1-[(2R,3R)-2-(2,5-difluorophenyl)-2-hydroxy-3-[4-(4-cyanophenyl)thiazol-2-yl]butyl]-1H-[1,2,4]triazol-4-ium chloride hydrochloride,
l) [[N-2-(methyl)phenyl-N-2- (acetoxy)ethyl]carbamoyloxy]methyl-1-[(2R,3R)-2-(2,5-difluorophenyl)-2-hydroxy-3-[4-(4-cyanophenyl)thiazol-2-yl]butyl]-1H-[1,2,4]triazol-4-ium iodide,
m) 1-[[N-2- [(isopropylamino) methyl]phenyl]carbamoyloxy]ethyl-1-[(2R,3R)-2-(2,5-difluorophenyl)-2-hydroxy-3-[4-(4-cyanophenyl)thiazol-2-yl]butyl]-1H-[1,2,4]triazol-4-ium chloride hydrochloride,
n) 1-[[ N-2-[ (pentan-3-ylamino) methyl]phenyl]carbamoyloxy]ethyl-1-[(2R,3R)-2-(2,5-difluorophenyl)-2-hydroxy-3-[4-(4-cyanophenyl)thiazol-2-yl]butyl]-1H-[1,2,4]triazol-4-ium chloride hydrochloride,
o) 1-[(N-methyl-N-2- [(methylamino) methyl]phenyl]carbamoyloxy]ethyl-1-[(2R,3R)-2-(2,5-difluorophenyl)-2-hydroxy-3-[4-(4-cyanophenyl)thiazol-2-yl]butyl]-1H-[1,2,4]triazol-4-ium chloride hydrochloride,
p) [[N-methyl-N-2-[ (methylamino)acetoxymethyl]phenyl]carbamoyloxy]methyl-1-[(2R,3R)-2-(2,5-difluorophenyl)-2-hydroxy-3-[4-(4-cyanophenyl)thiazol-2-yl]butyl]-1H-[1,2,4]triazol-4-ium chloride hydrochloride,
q) 1-[[N-methyl-N-2-[(methylamino)acetoxymethyl]phenyl]carbamoyloxy]ethyl-1-[(2R,3R)-2-(2,5-difluorophenyl)-2-hydroxy-3-[4-(4-cyanophenyl)thiazol-2-yl]butyl]-1H-[1,2,4]triazol-4-ium chloride hydrochloride,
r) 1-[[N-methyl-N-2- (methylamino)acetoxymethyl-4,5-difluorophenyl]carbamoyloxy]ethyl-1-[(2R,3R)-2-(2,5-difluorophenyl)-2-hydroxy-3-[4-(4-cyanophenyl)thiazol-2-yl]butyl]-1H-[1,2,4]triazol-4-ium chloride hydrochloride,
s) 1-[[N-methyl-N-2-(methylamino)acetoxymethyl-4-fluorophenyl]carbamoyloxy}ethyl-1-[(2R,3R)-2-(2,5-difluorophenyl)-2-hydroxy-3-[4-(4-cyanophenyl)thiazol-2-yl]butyl]-1H-[1,2,4]triazol-4-ium iodide hydrochloride,
t) [[N-methyl-N-2-(methylamino)acetoxymethyl-4,5-dimethoxyphenyl]carbamoyloxy]methyl-1-[(2R,3R)-2-(2,5-difluorophenyl)-2-hydroxy-3-[4-(4-cyanophenyl)thiazol-2-yl]butyl]-1H-[1,2,4]triazol-4-ium iodide hydrochloride,
u) 1-[(N-methyl-N-2- (methylamino)acetoxymethyl-5-fluorophenyl]carbamoyloxy]ethyl-1-[(2R,3R)-2-(2,5-difluorophenyl)-2-hydroxy-3-[4-(4-cyanophenyl)thiazol-2-yl]butyl]-1H-[1,2,4]triazol-4-ium chloride hydrochloride,
v) 1-[[N-methyl-N-2-(methylamino)acetoxymethyl-6-methylphenyl]carbamoyloxy]ethyl-1-[(2R,3R)-2-(2,5-difluorophenyl)-2-hydroxy-3-[4-(4-cyanophenyl)thiazol-2-yl]butyl]-1H-[1,2,4]triazol-4-ium iodide hydrochloride,
w) 1-[(N-methyl-N-2- (methylamino)acetoxymethyl-4-chlorophenyl]carbamoyloxy]ethyl-1-[(2R,3R)-2-(2,5-difluorophenyl)-2-hydroxy-3-[4-(4-cyanophenyl)thiazol-2-yl]butyl]-1H-[1,2,4]triazol-4-ium iodide hydrochloride,
x) 1-[[N- (methylamino)acetoxyethyl-N-2,4-difluorophenyl]carbamoyloxy]ethyl-1-[(2R,3R)-2-(2,5-difluorophenyl)-2-hydroxy-3-[4-(4-cyanophenyl)thiazol-2-yl]butyl]-1H-[1,2,4]triazol-4-ium chloride hydrochloride,
y) 1-[[N-methyl-N-2-(methylamino)acetoxymethyl-5-chlorophenyl]carbamoyloxy]ethyl-1-[(2R,3R)-2-(2,5-difluorophenyl)-2-hydroxy-3-[4-(4-cyanophenyl)thiazol-2-yl]butyl]-1H-[1,2,4]triazol-4-ium iodide hydrochloride,
z) 1-[[N-methyl-N-2- (methylamino)acetoxymethyl-5-nitrophenyl]carbamoyloxy]ethyl-1-[(2R,3R)-2-(2,5-difluorophenyl)-2-hydroxy-3-[4-(4-cyanophenyl)thiazol-2-yl]butyl]-1H-[1,2,4]triazol-4-ium chloride hydrochloride,
bb) 1-[[N-methyl-N-2- (methylamino)acetoxymethyl-3-fluorophenyl]carbamoyloxy]ethyl-1-[(2R,3R)-2-(2,5-difluorophenyl)-2-hydroxy-3-[4-(4-cyanophenyl)thiazol-2-yl]butyl]-1H-[1,2,4]triazol-4-ium chloride hydrochloride,
cc) 1-[[N-methyl-N-2- (methylamino)acetoxymethyl-5-cyanophenyl]carbamoyloxy]ethyl-1-[(2R,3R)-2-(2,5-difluorophenyl)-2-hydroxy-3-[4-(4-cyanophenyl)thiazol-2-yl]butyl]-1H-[1,2,4]triazol-4-ium chloride hydrochloride,
dd) 1-([N-methyl-N-2- (methylamino)acetoxymethyl-3-chlorophenyl]carbamoyloxy]ethyl-1-[(2R,3R)-2-(2,5-difluorophenyl)-2-hydroxy-3-[4-(4-cyanophenyl)thiazol-2-yl]butyl]-1H-[1,2,4]triazol-4-ium chloride hydrochloride,
ee) 1-[[N-methyl- N-2- (methylamino)acetoxymethyl-4-cyanophenyl]carbamoyloxy]ethyl-1-[(2R,3R)-2-(2,5-difluorophenyl)-2-hydroxy-3-[4-(4-cyanophenyl)thiazol-2-yl]butyl]-1H-[1,2,4]triazol-4-ium chloride hydrochloride,
ff) 1-[[N-methyl-N-2- (methylamino)acetoxymethyl-5-trifluoromethylphenyl]carbamoyloxy]ethyl-1-[(2R,3R)-2-(2,5-difluorophenyl)-2-hydroxy-3-[4-(4-cyanophenyl)thiazol-2-yl]butyl]-1H-[1,2,4]triazol-4-ium iodide hydrochloride,
gg) 1-[(N-methyl-N-2- (amino)acetoxymethyl-3-chlorophenyl]carbamoyloxy]ethyl-1-[(2R,3R)-2-(2,5-difluorophenyl)-2-hydroxy-3-[4-(4-cyanophenyl)thiazol-2-yl]butyl]-1H-[1,2,4]triazol-4-ium chloride hydrochloride,
hh) 1-[[N-ethyl-N-2-(methylamino)acetoxymethyl-3-chlorophenyl]carbamoyloxy]ethyl-1-[(2R,3R)-2-(2,5-difluorophenyl)-2-hydroxy-3-[4-(4-cyanophenyl)thiazol-2-yl]butyl]-1H-[1,2,4]triazol-4-ium chloride hydrochloride,
ii) 1-[[N-methyl-N-3- [(amino)acetoxymethyl]pyridin-2-yl]carbamoyloxy]ethyl-1-[(2R,3R)-2-(2,5-difluorophenyl)-2-hydroxy-3-[4-(4-cyanophenyl)thiazol-2-yl]butyl]-1H-[1,2,4]triazol-4-ium chloride hydrochloride,
jj) 1-[[N-methyl-N-2- (methylamino)acetoxymethyl-3-methylphenyl]carbamoyloxy]ethyl-1-[(2R,3R)-2-(2,5-difluorophenyl)-2-hydroxy-3-[4-(4-cyanophenyl)thiazol-2-yl]butyl]-1H-[1,2,4]triazol-4-ium iodide hydrochloride,
kk) 1-[[N-ethoxycarbonyl-N-2- (methylamino)acetoxymethyl-phenyl] carbamoyloxy]ethyl-1-[(2R,3R)-2-(2,5-difluorophenyl)-2-hydroxy-3-[4-(4-cyanophenyl)thiazol-2-yl]butyl]-1H-[1,2,4]triazol-4-ium chloride hydrochloride,
ll) 1-[[N-pivaloyl-N-2- (methylamino)acetoxymethyl-phenyl] carbamoyloxy]ethyl-1-[(2R,3R)-2-(2,5-difluorophenyl)-2-hydroxy-3-[4-(4-cyanophenyl)thiazol-2-yl]butyl]-1H-[1,2,4]triazol-4 -ium chloride hydrochloride.
and
pharmaceutically acceptable salts, hydrates or solvates thereof.

The most preferred compound is 1-[[N-methyl-N-3-[(methylamino)acetoxymethyl]pyridin-2-yl]carbamoyloxy]ethyl-1-[(2R,3R)-2-(2,5-difluorophenyl)-2-hydroxy-3-[4-(4-cyanophenyl)thiazol-2-yl]butyl]-1H-[1,2,4]triazol-4-ium chloride hydrochloride and pharmaceutically acceptable salts, hydrates or solvates thereof.

A further embodiment of the present invention is directed to intermediates useful for the preparation of the above-defined compounds. Preferred intermediates are compounds of formula (V) 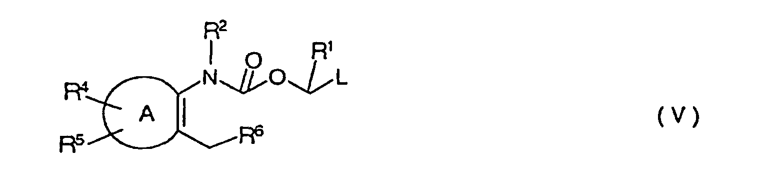 wherein R¹, R², R⁴, R⁵, R⁶ and the group 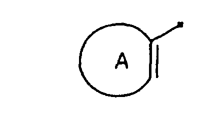 are as defined above and L is a leaving group.

Preferred compounds of formula (V) are compounds selected from the group consisting of
a) [N-methyl-N-2-(acetoxymethyl) phenyl]carbamic acid chloromethyl ester,
b) [N-methyl-N-2- ((tert-butoxycarbonylisopropylamino) methyl) phenyl]carbamic acid 1-chloro-ethyl ester,
c) [N-methyl-N-3-((tert-butoxycarbonylmethylamino)acetoxymethyl) pyridin-2-yl]carbamic acid 1-chloro-ethyl ester,
e) [N-methyl-N-phenyl]carbamic acid chloromethyl ester,
f) [N-methyl-N-3-(acetoxymethyl)pyridin-2-yl]carbamic acid 1-chloro-ethyl ester,
k) [N-2-(methyl)phenyl-N-acetoxyethyl]carbamic acid chloromethyl ester,
l) [ N-2- [(tert-butoxycarbonylisopropylamino) methyl]phenyl]carbamic acid 1-chloro-ethyl ester,
m) [N-2- [(tert-butoxycarbonyl-pentan-3-ylamino) methyl]phenyl]carbamic acid 1-chloro-ethyl ester,
n) [N-methyl-N-2-[(tert-butoxycarbonylmethylamino) methyl]phenyl]carbamic acid 1-chloro-ethyl ester,
o) [N-methyl-N-2-[(tert-butoxycarbonyl methylamino)acetoxymethyl]phenyl]carbamic acid chloromethyl ester,
p) [N-methyl-N-2-[ (tert-butoxycarbonyl methylamino)acetoxymethyl]phenyl]carbamic acid 1-chloro-ethyl ester,
q) [N-methyl-N-2-[(tert-butoxycarbonylmethylamino)acetoxymethyl]-4,5-difluoro-phenyl]carbamic acid 1-chloro-ethyl ester,
r) [N-methyl-N-2- [(tert-butoxycarbonylmethylamino)acetoxymethyl]-4-fluoro-phenyl]carbamic acid 1-chloro-ethyl ester,
s) [N-methyl-N-2-[(tert-butoxycarbonylmethylamino)acetoxymethyl]-4,5-dimethoxy-phenyl]carbamic acid chloromethyl ester,
t) [N-methyl-N-2-[(tert-butoxycarbonylmethylamino)acetoxymethyl]-5-fluoro-phenyl]carbamic acid 1-chloro-ethyl ester,
u) [N-methyl-N-2- [(tert-butoxycarbonylmethylamino)acetoxymethyl]-6-methyl-phenyl]carbamic acid 1-chloro-ethyl ester,
v) [N-methyl-N-2- [(tert-butoxycarbonylmethylamino)acetoxymethyl]-4-chloro-phenyl]carbamic acid 1-chloro-ethyl ester,
w) [N-(tert-butoxycarbonylmethylamino)acetoxyethyl-N-2,4-difluorophenyl]carbamic acid 1-chloro-ethyl ester,
x) [N-methyl-N-2- [(tert-butoxycarbonylmethylamino)acetoxymethyl]-5-chloro-phenyl]carbamic acid 1-chloro-ethyl ester,
y) [N-methyl-N-2-[(tert-butoxycarbonylmethylamino)acetoxymethyl]-5-nitro-phenyl)carbamic acid 1-chloro-ethyl ester,
aa) [N-methyl-N-2-[(tert-butoxycarbonylmethylamino)acetoxymethyl]-3-fluoro-phenyl]carbamic acid 1-chloro-ethyl ester,
bb) [N-methyl-N-2-[(tert-butoxycarbonylmethylamino)acetoxymethyl]-5-cyano-phenyl]carbamic acid 1-chloro-ethyl ester,
cc) [N-methyl-N-2-[(tert-butoxycarbonylmethylamino)acetoxymethyl]-3-chloro-phenyl]carbamic acid 1-chloro-ethyl ester,
dd) [N-methyl-N-2-[(tert-butoxycarbonylmethylamino)acetoxymethyl]-4-cyano-phenyl)carbamic acid 1-chloro-ethyl ester,
ee) [N-methyl-N-2-[(tert-butoxycarbonylmethylamino)acetoxymethyl]-5-trifluoromethyl-phenyl)carbamic acid 1-chloro-ethyl ester,
ff) [N-methyl-N-2-[(tert-butoxycarbonylamino)acetoxymethyl]-3-chlorophenyl)carbamic acid 1-chloro-ethyl ester,
gg) [N-ethyl-N-2-[(tert-butoxycarbonylmethylamino)acetoxymethyl]-3-chloro-phenyl]carbamic acid 1-chloro-ethyl ester,
hh) [N-methyl-N-3-[(tert-butoxycarbonylamino)acetoxymethyl]pyridin-2-yl]carbamic acid 1-chloro-ethyl ester,
ii) [N-methyl-N-2-[(tert-butoxycarbonylmethylamino)acetoxymethyl]-3-methyl-pheny]carbamic acid 1-chloro-ethyl ester,
jj) [N-ethoxycarbonyl-N-2-((tert-butoxycarbonylmethylamino) acetoxy methyl)pheny]carbamic acid 1-chloro-ethyl ester,
kk) [N-pivaloyl-N-2-((tert-butoxycarbonylmethylamino)acetoxymethyl)phenyl]carbamic acid 1-chloro-ethyl ester,

The compounds of formula (II) are well known azole antifungals and commercially available (e.g. Prepn. of 1-[2-(2,4-Dichlorophenyl)-2-[(2,4-dichlorophenyl)methoxy]ethyl]-1H-imidazole: E. F. Godefroi et al., J. Med. Chem. 12,784 (1969); Prepn. of cis-1-Aceryl-4-[4-[[2-(2,4-dichlorophenyl)-2-(1H-imidazol-1-ylmethyl)-1,3-dioxolan-4- yl]methoxy]phenyl]piperazine: J. Heeres et al., Ger. Pat. 2,804,-096; Prepn. of 4-[4-[4-[4-[[2-(2,4-Dichlorophenyl)-2-(1H-1,2,4-triazol-1-ylmethyl)-1,3-dioxolan-4-yl]methoxy]phenyl]-1-piperazinyl]phenyl]-2,4-dihydro-2-(1-methylpropyl)-3H-[1,2,4]triazol-3-one: J. Heeres, L. J. J. Backx, Eur. Pat.Appl.6,711; Prepn. of 2-[(1R,2R)-2-(2,4-diflorophenyl)-2-hydroxy-1-methyl-3-(1H-1,2,4-triazol-1-yl)propyl]-4-[4-(2,2,3,3-tetrafluoropropoxy)phenyl]-3(2H,4H)-1,2,4-triazolone: A. Tasaka et al., Chem. Pharm. Bull. 45,321-326,1997; Prepn. of (+)-2-(2,4-Difluorophenyl)-3-methyl-1-(1H-1,2,4-triazol-1-yl)-3-(6-(1H-1,2,4-triazol-1-yl)pyridazin-3-ylthio)butan-2-ol: T. Kai et al., Chem. Pharm. Bull. 44 (3),568-571, 1996; Prepn. of (2R)-2-(2,4-diflourophenyl)-1-[3-[(E)-4-(2,2,3,3-tetrafluoropropoxy)-styryl]-(1,2,4-triazol-1-yl)-3-(1,2,4-triazol-1-yl)]propan-2-ol: H. Yamada et al., Antimicrob. Agents Chemother. 37 (11), 2412-2417, 1993; Prepn. of dl-Threo-2-(2,4-difluorophenyl)-3-methyl-sulfonyl-1-(1H-1,2,4-triazol-1-yl)-butan-2-ol: Drugs Future, 17, 1145-1146, 1992; Prepn. of (-)-4-[4-[4-[4-[[5-(2,4-Difluorophenyl)-5-(1H-1,2,4-triazol-1-ylmethyl)tetrahydrofuran-3-yl]methoxy]phenyl]piperazinyl]phenyl]-2[(1S,2S)-1-ethyl-2-hydroxypropyl]-3H-1,2,4-triazol-3-one: Curr. Pharm. Des., 2, 209-224, 1996; Prepn. of (2R,3R)-3-[4-(4-cyanophenyl)thiazol-2-yl)]-2-(2,4-difluorophenyl)-1-(1H-1,2,4-triazol-1-yl)-butan-2-ol: Eisai Co. Ltd., EP 667346; Prepn. of 3-Methyl-3-methylthio-1-(1,2,4-triazol-1-yl)-2-(trifluoromethylphenyl)-butan-2-ol: S. S. Pharmaceutical Co. Ltd., EP 435081.

Other azoles of formula (II) as well as salts, hydrates or solvates thereof, like 3-fluoro, 2,5-difluoro- and 2,4,5-trifluoro-derivatives, can be manufactured according to the following synthetic scheme A, starting from 4-[(2R)-2-(3,4,5,6-tetrahydro-2H-pyran-2-yloxy)propionyl]morpholine [which can be prepared by a same procedure as described in Chem. Pharm. Bull. 41, 1035, 1993.]. This synthesis route has been described for example in European Patent Application No. 99101360.8. 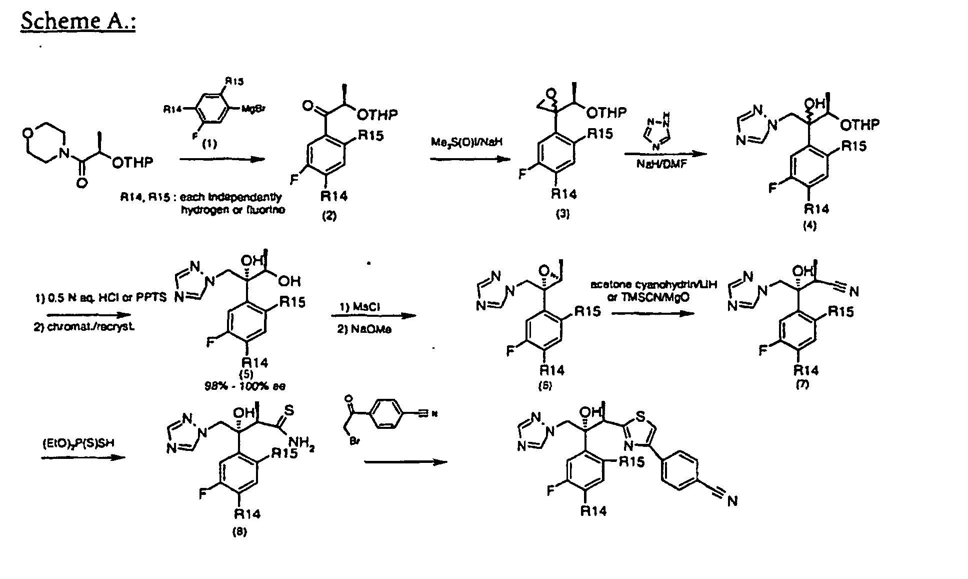
(a) Reacting 4-[(2R)-2-(3,4,5,6-tetrahydro-2H-pyran-2-yloxy)propionyl] morpholine with a compound of the formula (1) in an organic solvent such as tetrahydrofuran (THF) at a temperature between -10°C and room temperature for 3 to 8 hr. to give a compound of the formula (2), 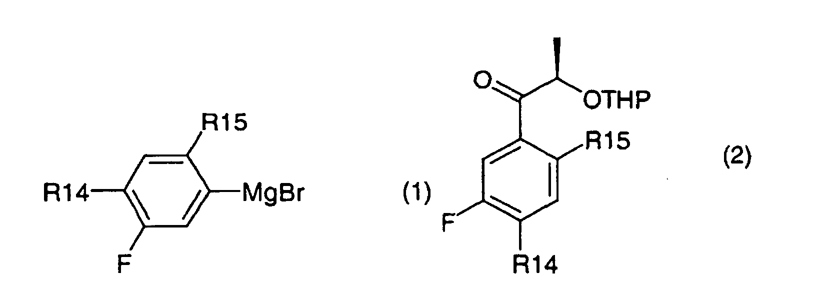 in which R¹⁴ and R¹⁵ are each independently for example hydrogen or fluorine (hereinafter R¹⁴ and R¹⁵ have the same meaning),
   followed by
(b) reacting a compound of the formula (2) with trimethyl sulfoxonium iodide, in the presence of sodium hydride in THF and dimethyl sulfoxide (DMSO) or in the presence of BuLi in THF and N,N'-dimethylpropylene urea (DMPU), at a temperature between -5°C and room temperature for 2 to 8 hr. to give a compound of the formula (3), 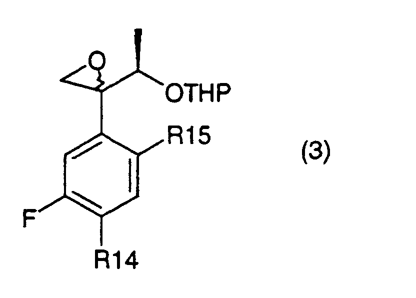 followed by
(c) reacting a compound of the formula (3) with triazole in the presence of sodium hydride in dry dimethylformamide (DMF) at a temperature between 50°C and 100°C for 6 to 12 hr. to give a compound of the formula (4), 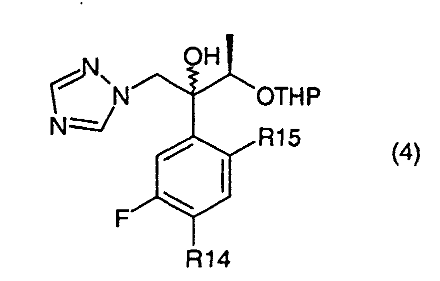 followed by
(d) reacting a compound of the formula (4) with aqueous hydrochloric acid at a concentration between 1N and 0.1N solution, in methanol and n-hexane at room temperature or pyridinium p-toluenesulfonate in ethanol, at a temperature between room temperature and 100°C for 2 to 6 hr. The resulting compound is recrystalized from t-butyl methyl ether and n-hexane to give a compound of the formula (5), 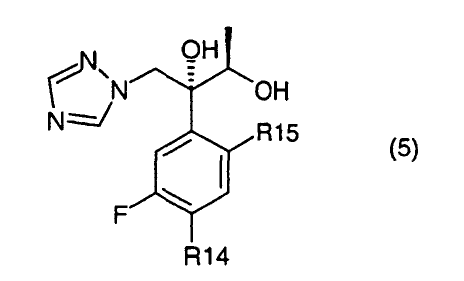 followed by
(e) reacting a compound of the formula (5) with mesyl chloride in CH₂Cl₂ and methyl acetate (AcOEt) in the presence of an organic base such as triethylamine or pyridine for 30 min. to 2 hr. This reaction is followed by epoxy ring formation with sodium methoxide in methanol for 15 min. to 1 hr. The resulting compound is purified by recrystalization from t-butyl methyl ether and n-hexane or by silicagel column chromatography using CH₂Cl₂ and methanol as eluent, to give a compound of the formula (6), 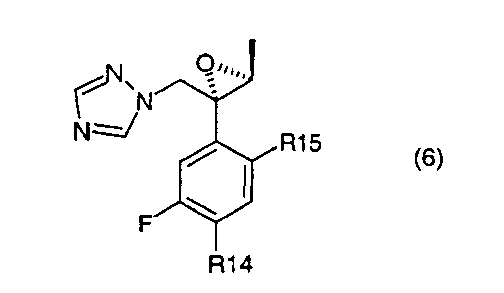 followed by
(f) reacting a compound of the formula (6) with acetone cyanohydrin in the presence of lithium hydride in THF under reflux for 4 to 8 hr.or trimetylsilyl cyanide in the presence of magnesium oxide in o-xylene at a temperature between 100°C and 160°C for 20 to 40 hr, then removing of trimethylsilyl group with conc. hydrogen chloride solution in THF to give a compound of the formula (7), 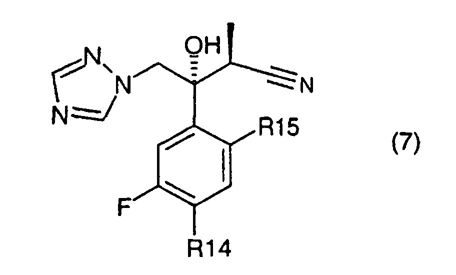 followed by
(g) reacting a compound of the formula (7) with dithiophosphoric acid O,O-diethyl ester and water or dithiophosphoric acid O,O-diethyl ester, water and iso-propanol at a temperature between 90°C and 150°C for 4 to 8 hr. to give a compound of the formula (8). 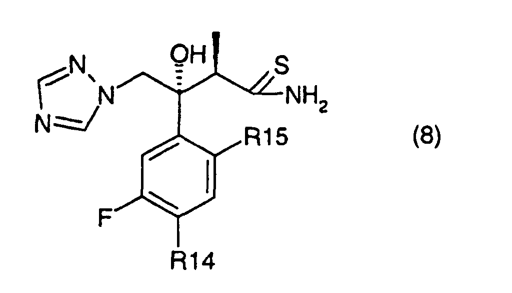 followed by
(h) reacting a compound of the formula (8) with 2-bromo-4'-cyanoacetophenone at a temperature between room temperature and 80°C in acetonitrile, ethanol or methanol for 2 to 24 hr. to give a compound of the formula (II),

The compounds of the formula (IV) and (V) can be prepared by procedures similar to those known in the art. The typical example of the reaction is disclosed in Example 1[scheme(1)], Example 2[scheme(2)], Example 3[scheme (3)], or Example 4[scheme(4)]. In these examples, each starting material[(a), (d), (i), (n) or (r)] was purchased from TOKYO CHEMICAL INDUSTRY CO., LTD (1-13-6 Nihonbashi Muromachi, Chuo-ku, Tokyo 103, Japan). Other starting materials are known in the art and/or commercially available.

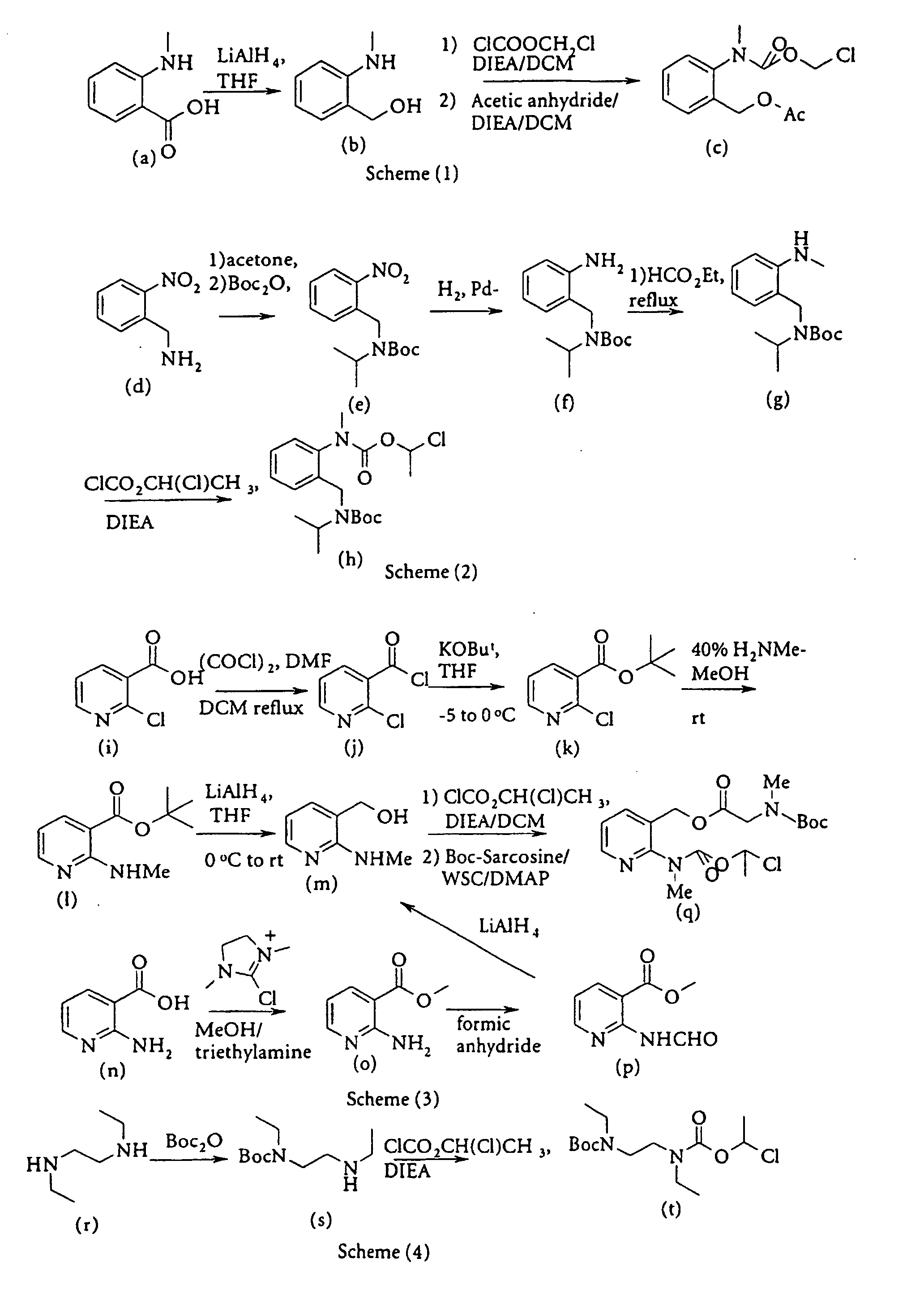

Accordingly, the present invention also refers to a process for the manufacture of a compound of formula (III) as defined above as well as salts, hydrates ofsolvates thereof, which comprises reacting an azole compound possessing antifungal activity of the general formula (II) as defined above with a compound of general formula (V) as defined above.

The typical example of the reaction of a compound of the formula (II) with the compound of the formula (V) is disclosed in Example 5[scheme(5)], Example 6[scheme(6)] or Example 7[scheme(7)]. In these Examples, the compounds were synthesized by procedures known to those skilled in the art which are described for example in European Patent Application No.99101360.8.

The quarternarization reaction can be carried out in a solvent such as methylene chloride, chloroform, benzene, toluene, acetonitrile, tetrahydrofuran, dioxane, or dimethylformamide, preferably chloroform, acetonitrile, or dimethylformamide.

The reaction time in the above quarternarization reaction may be varied within a relatively wide range. In general, the reaction can be carried out at a temperature between 0°C and 100°C, preferably between 0°C and 50°C.

Preferably, an amino group present in R⁶ in the compound of formula (V) are protected by a suitable amino protecting group as tert-butoxy carbonyl.

The protecting group may, if necessary, be removed after the quarternarization reaction as disclosed in Example 6[scheme(6)] or Example 7[scheme(7)] by procedures known to those skilled in the art.

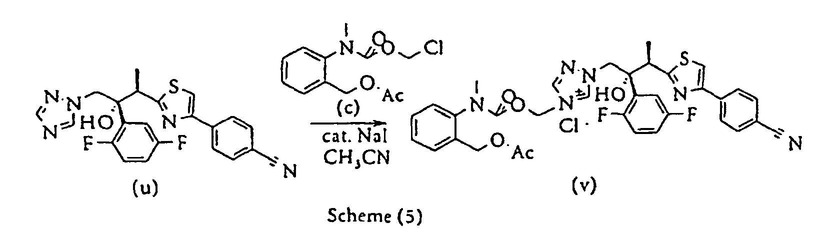

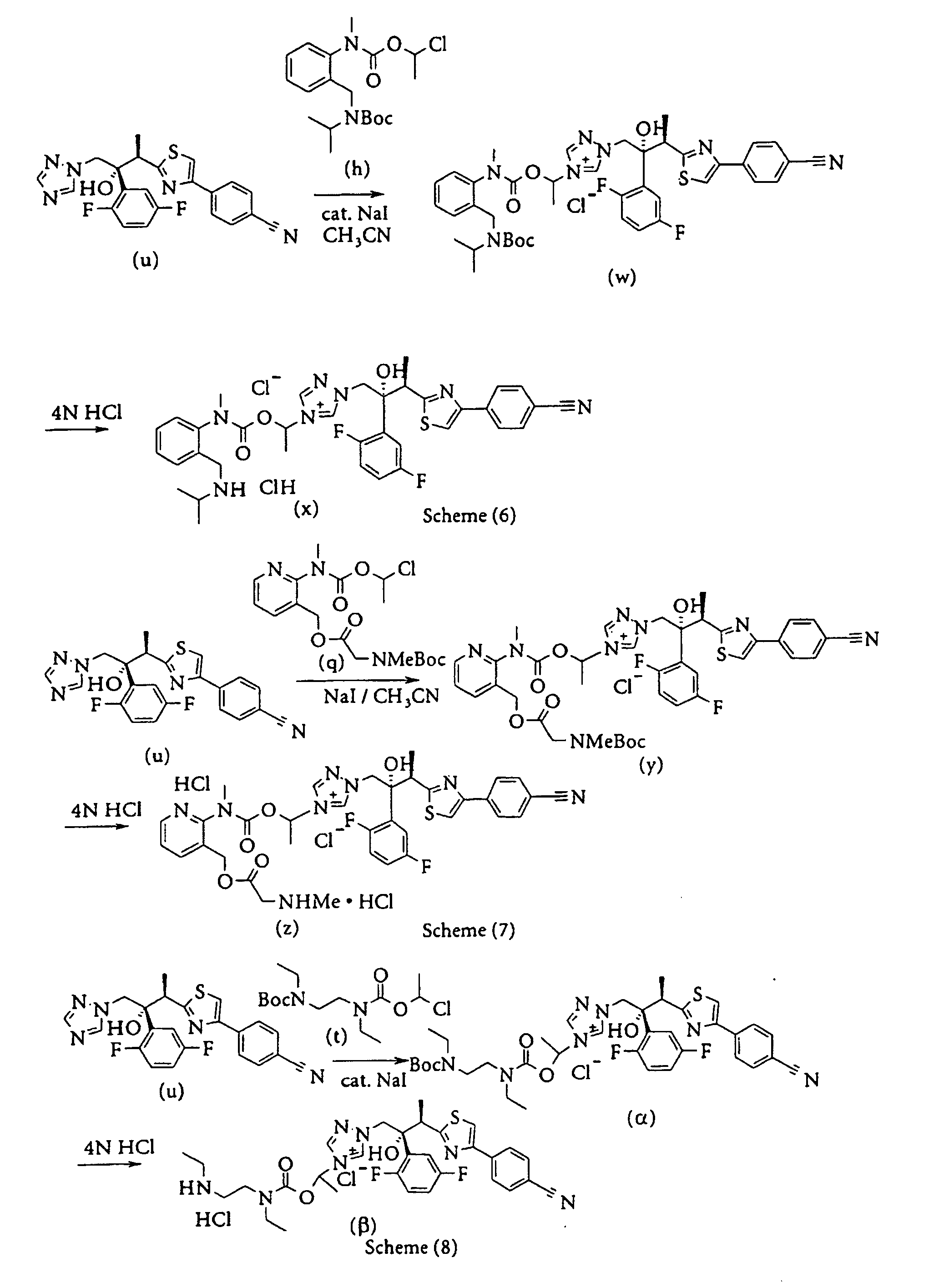

The compounds of the formula (III) may contain an amino acid ester substituent and/or other basic amino groups which may form acid addition salts. The term "salts of compounds of the formula (III)" refers to such acid addition salts. These salts may be derived from pharmaceutically acceptable acids as described earlier with reference to the Symbol X⁻. The salt formation can be performed when removing a protecting group, or can be performed ad hoc by procedures known per se.

The hydration can be effected in the course of the manufacturing process or can occur gradually as a result of hygroscopic properties of an initially anhydrous product. Solvates with pharmaceutically acceptable solvents such as ethanol can be obtained for example, during precipitation.

The present invention also refers to the above compounds of formula (III) as obtained by a process as described above and to a pharmaceutical composition, in particular for use as an antifungal, comprising a compound as defined above and a pharmaceutically acceptable carrier.

Further the present invention is directed to a method of treating fungal infections comprising administering to the infected organism an effective amount of a compound as defined above, to the compounds as defined above for use as therapeutic active substances, in particular as antifungallly active substances, and to the use of a compound as defined above for the preparation of a medicament for the prophylaxis and/or treatment of fungal infections. Such a medicament comprises a compound as defined above.

The novel azole compounds represented by the formula (III) as well as hydrates or solvates thereof have much higher water solubility than known antimycotic azole compounds represented by the formula (II) (see Table 1).

**Table 1:**

| Solubility | | |
|---|---|---|
| Compound (Example No.) | Solubility (mg/ml) | Solvent |
| 5 | 1 | a |
| 6 | >10 | a |
| 7 | >1000 | a, b |
| 6.1. | >10 | a |
| 6.2. | >10 | a |
| 7.10. | >10 | a |
| 7.15. | >10 | a |
| 7.20. | >30 | a |
| 7.21. | >10 | a |
| * solvent a = distilled water, solvent b = physiological saline | | |

In addition, the novel azole compounds of the formula (III) are chemically stable in aqueous solution at room temperature more than three days, but are efficiently converted into compounds of the formula (II) in either mouse, rat, monkey or human plasma.

The conversion of representatives of the new azole compounds of the formula (III) to (2R,3R)-3-[4-(4-cyanophenyl) thiazol-2-yl)]-2-(2,5-difluorophenyl)-1-(1H-1,2,4-triazol-1-yl)-butan-2-ol, in human plasma are shown in Table 2.

The compounds of formula were incubated with human plasma at a concentration of 10µg/ml at 37°C for up to 120 min. After quenching by the addition of EtOH, conversion half-life was determined by HPLC-MASS analysis(see Example D).

**Table 2:**

| Conversion of the new azole compounds to (2R,3R)-3-[4-(4-cyanophenyl)thiazol-2-yl)]-2-(2,5-difluorophenyl)-1-(1H-1,2,4-triazol-1-yl)-butan-2-ol(u) in plasma | |
|---|---|
| Example No | Cnversion to (2R,3R)-3-(4-(4-cyanophenyl)thiazol-2-yl)]-2-(2,5-difluorophenyl)-1-(1H-1,2,4-triazol-1-yl)-butan-2-ol( u) in plasma |
| 5 | < 2min in rat plasma |
| 7 | < 2min in human plasma |
| 7.3. | 6 min in human plasma |
| 7.4. | 2 min in human plasma |
| 7.10. | 3 min in human plasma |
| 7.13. | 2 min in human plasma |

In vivo efficacy of the compounds of the present invention is shown in table 3. Male Fisher rats, strain F344/DuCrj, were employed for experimental infection models such as systemic candidiasis, systemic aspergillosis and pulmonary aspergillosis model. Immunocompetent 4 weeks old rats were used for systemic candidiasis or systemic aspergillosis which occurred after infection with Candida albicans conidia of 5x10⁶/rat or with Aspergillus fumigatus conidia of 6x10⁵/rat via tail vein. Otherwise for pulmonary aspergillosis model, rats had been immunosuppressed with cortisone acetate treatments prior to infection with 2x10⁵/rat intratrachially. Treatments were given twice on the first day and once daily on following 4 days both for systemic and pulmonary aspergillosis (Ib.i.+4q.d.), for systemic candidiasis rats were treated at 0, 4, 24, and 48 h after infection (Ib.i.d.+2q.d.). Effective dose 50% (ED50) values were determined on day 14 after infection.

**Table:**

| in vivo efficacy | | | | | | |
|---|---|---|---|---|---|---|
| (µmol/kg) | | | | | | |
| | Systemic Fluconazole resistance candidiasis | | Pulmonay aspergillosis | | Systemic aspergillosis | |
| | p.o. | i.v. | p.o. | i.v. | p.o. | i.v. |
| Example 7 | 14.1 | 13.3 | | | 8.8 | 13.5 |
| | | | | | | |
| Itraconazole Fluconazole | 21.8 | | | | 4.9 | |

| **Rat systemic mycosis[ED50(µmol / kg) on day14]** | | | | | | | |
|---|---|---|---|---|---|---|---|
| | | *C. albicans* CY1002 | *C. albicans* CY3003 | *C. parapsilosis* KULM219C | *C. tropicalis* CY5042 | FCZ^{R}. *C. albicans* UTHS93-2067 | *A. lumigatus* CF1003 |
| Example 7 | I.V. | 4 | 1.6 | 7.6 | 1 | 2.8 | 11 |
| | PO. | 4 | 2.5 | 6.5 | 1 | 7 | 9 |
| Fluconazole | PO. | 1.9 | 1 | 17 | 2 | 8.1 | 94 |
| Itraconazole | PO. | 4.7 | 2.3 | 20 | 2 | 4.2 | 8.9 |

Therefore, the water soluble azole antifungal agents, represented by the formula (III) as well as salts, hydrates or solvates thereof, according to the present invention, exhibit potent antifungal activity against various fungal infections including Aspergillosis in mice over a very wide range of dosages both orally and parenterally and are useful as antifungal agents.
The present invention further relates to the pharmaceutical compositions containing the azole compound of the formula (III) as well as salts, hydrates or solvates thereof and pharmaceutically acceptable carrier.

The azole compounds of the formula (III) as well as salts, hydrates or solvates thereof are active against a variety of fungal species including *Candida spp., Cryptotoccus neoformans, Aspergillus spp., Trichophyton* spp., *Microsporum* spp., *Exophiala* spp., *Blastomyces dermatitidis,* and *Histoplasma capsulatum*.

Thus, the compounds of the present invention are useful for topical and systemic treatment of mycoses in animals as well as in humans. For example, they are useful in treating topical and mucosal fungal infections caused by, among other genera, *Candida, Trichophyton*, or *Microsporum.* They may also be used in the treatment of systemic fungal infections caused by, for example, *Candida spp., Cryptococcus neoformans, Aspergillus spp., Paracoccidiodes spp., Sporotrix spp., Exophiala spp., Blastomyces spp.,* or *Histoplasma spp..*

For clinical use, the azole compounds of the formula (III) as well as salts, hydrates or solvates thereof can be administered alone, but will generally be administered in pharmaceutical admixture formulated as appropriate to the particular use and purpose desired, by mixing excipient, binding agent, lubricant, disintegrating agent, coating material, emulsifier, suspending agent, solvent, stabilizer, absorption enhancer and/or ointment base. The admixture can be used for oral, injectable, rectal or topical administration.

Pharmaceutical formulation for oral administration may be granule, tablet, sugar coated tablet, capsule, pill, suspension or emulsion. For parenteral injection, for example, intravenously, intramuscularly or subcutaneously, the azole compounds of formula (III) may be used in the form of a sterile aqueous solution which may contain other substances, for example, salts or glucose to make the solution isotonic. The azole compounds can also be administered in the form of a suppository or pessary, or they may be applied topically in the form of a lotion, solution, cream, ointment or dusting powder.

The daily dosage level of the azole compounds of the formula (III) is from about 0.1 to about 50 mg/kg (in divided doses) when administered in one, two or more dosages by either the oral or parenteral route. Thus tablets or capsules of the compounds may contain from about 5 mg to about 0.5 g of active compound for administration. In any event the actual dosage can be determined by the physician and it may be varied upon the age, weight and response of the particular patient.

In addition, the azole compounds of the formula (III) as well as salts, hydrates or solvates thereof have activity against a variety of plant pathogenic fungi, including for example *Pyricularia oryzae, Pythium aphanidermatum, Alternaria* spp., and *Paecilomyces variotii.*

Thus, they can be applied for agricultural and horticultural purposes preferably in the form of a composition formulated as appropriate to the particular use and purpose desired, for example dusting powders, or granules, seed dressings, aqueous solutions, dispersions or emulsions, dips, sprays or aerosols. Such compositions may contain such conventional carriers, diluents or adjuvants as are known and acceptable in agriculture and horticulture. Other compounds having herbicidal or insecticidal, or additional antifungals can be incorporated in the compositions. The compounds and compositions can be applied in a number of ways, for example they can be applied directly to the plant foliage, stems, branches, seeds or roots or to the soil or other growing medium, and they may be used not only to eradicate the disease, but also prophylactically to protect the plants or seeds from attack.

The following examples illusirate the preferred methods for the preparation of the compounds of the present invention, which are not intended to limit the scope of the invention thereto.

### EXAMPLES

### Example 1

### [N-methyl-N-2-(acetoxymethyl) phenyl]carbamic acid chloromethyl ester

### a) Preparation of 2-(N-methylamino)-benzylalcohol

To a suspension of lithium aluminum hydride (0.76 g, 0.02 mol) in dry tetrahydrofuran (40 ml) was added a solution of N-methylanthranilic acid(a) (1.51 g, 0.01 mol) in dry tetrahydrofuran under Ar atmosphere. After refluxing for 1h, the reaction was quenched by adding ice water (50 ml). The mixture was filtered on a celite pad and thoroughly washed with dichloromethane (50 ml). The organic layer was separated and the water layer was extracted with dichloromathane (50 ml). The combined organic layer was washed with brine (30 ml), dried over anhydrous sodium sulfate and concentrated under reduced pressure. The resulting yellowish oil was purified on a column of silica gel (wakogel C-200 50 g, eluent n-hexane: ethyl acetate = 2:1) to give the title compound(b) as colorless oil (1.18 g, 86 %). ¹H-NMR (270 MHz, CDCl₃): δ 2.87 (3H, s), 3.00-3.10 (1H, br.s), 4.64 (2H, s), 6.65-6.69 (2H, m), 7.05 (1H, d, J = 7.2), 7.23-7.29 (1H, m)

### b) Preparation of [N-methyl-N-2-(acetoxymethyl) phenyl]carbamic acid chloromethyl ester

### Step 1

To a solution of 2-(N-methylamino)-benzylalcohol(b) (536 mg, 3.9 mmol) in dry dichloromethane (25 ml) and diisopropylethylamine (681 µl, 3.9 mmol)was added dropwise chloromethyl chloroformate (360 µl, 4.0 mmol)and the reaction mixture was stirred at 0 °C with occasional check of the reaction progress by t.l.c (n-hexane:ethyl acetate = 2:1). After 2h, the starting material disappeared on t.l.c and the solution was used directly for the following reaction.

### Step 2

To the reaction mixture were added diisopropylethylamine (900 µl, 5.0 mmol) and acetic anhydride (400 mg) and stirred for 3 h at ambient temperature. The reaction mixture was partitioned with dichloromethane (50ml) and water (30 ml). The water layer was separated and extracted again with dichloromethane (50ml). The combined organic layer was washed with brine (30 ml x 2), dried over anhydrous sodium sulfate and concentrated under reduced pressure. The resulting yellowish oil was purified on a column of silica gel (wakogel C-200 10 g, eluent dichloromethane: methanol = 200:1) to give the title compound(c) as colorless syrup (740 mg, 70 %). EI-MS: m/z 271(M⁺); ¹H-NMR (270 MHz, CDCl₃): δ 2.10 (3H, s), 3.30 (3H, s), 5.07-5.78 (2H, br.d), 5.60 (0.8H, d, J = 5.9), 5.73 (0.8H, d, J = 5.9), 5.85 (0.4H, br.s), 7.16-7.23 (1H, m), 7.37-7.49 (3H, m).

The following compounds in Example 1.1. - 1.7. were obtained according to a manner analogous to those of Example 1.

### 1.1.

[N-methyl-N-phenyl]carbamic acid chloromethyl ester. Physical form: colorless oil; LC-MS: m/z200(M+1)⁺; ¹H-NMR(CDCl₃): δ 3.35 (s, 3H), 5.76 (bs, 2H), 7.19-7.43 (m, 5H).

### 1.2.

[N-methyl-N-3-(acetoxymethyl)pyridin-2-yl]carbamic acid 1-chloro-ethyl ester. Physical form: colorless oil; LC-MS: m/z287(M+1)⁺; ¹H-NMR(CDCl₃): δ 1.60(3H,br.s), 2.12(3H,s), 3.35(3H,br.s), 5.10(2H,m), 6.57(1H,m), 7.30(1H,m), 7.82(1H,m), 8.46(1H,m).

### 1.7.

[N-2-(methyl)phenyl-N-acetoxyethyl]carbamic acid chloromethyl ester. Physical form: yellow oil; LC-MS: m/z286(M+1)⁺; ¹H-NMR (CDCl₃): δ 1.99 (s, 3H), 2.24 (s, 3H), 3.62-3.69 (m, 1H), 4.04-4.29 (m, 3H), 5.58 (d, J = 5.9 Hz, 1H), 5.79 (d, j = 5.9 Hz, 1H), 7.12-7.28 (m, 4H).

### Example 2

### (N-methyl-N-2- ((tert-butoxycarbonylisopropylamino) methyl) phenyl)carbamic acid 1-chloro-ethyl ester

### a) Preparation of Isopropyl-(2-nitro-benzyl)-carbamic acid tert-butyl ester

To a mixture of 2-nitrobenzylamine hydrochloride (500 mg, 2.65 mmol) and acetone (0.39 ml, 5.30 mmol) in methanol (13 ml) was added sodium cyanoborohydride (500 mg, 7.95 mmol) at 0°C. The temperature was warm up to room temperature. After stirring for 3hr, the mixture was concentrated in vacuo and extracted with dichloromethane. The combined organic phase was washed with water and brine, dried over anhydrous magnesium sulfate, filtered and concentrated in vacuo to give N-isopropyl-2-nitrobenzylamine as yellow oil. This compound was used in next step without further purification.

To a mixture of N-isopropyl-2-nitrobenzylamine and N,N-diisopropylethyl amine (1.15ml, 6.63mmol) in tetrahydrofuran (20ml) was added di-tert-butyl dicarbonate (1.22ml, 5.30mmol) at room temperature. After stirring overnight, the mixture was quenched with water and extracted with ethyl acetate. The combined organic phase was washed with water and brine, dried over anhydrous magnesium sulfate, filtered and concentrated in vacuo. The residue was purified by column chromatography (15% ethyl acetate-hexane) to afford isopropyl-(2-nitro-benzyl)-carbamic acid tert-butyl ester (e) (736mg, 2.50mmol, 94%) as light yellow oil.

### b) Preparation of (2-Amino-benzyl)-isopropyl--carbamic acid tert-butyl ester

To a solution of isopropyl-(2-nitro-benzyl)-carbamic acid tert-butyl ester (730 mg, 2.48 mmol) in ethyl acetate (10 ml) was added acetic acid (0.156 ml, 2,73 mmol) and catalytic amount of palladium 10wt.% on activated carbon. The mixture was stirred overnight under hydrogen atmosphere. The mixture was filtered and concentrated in vacuo. The residue was purified by column chromatography (15% ethyl acetate-hexane) to afford (2-amino-benzyl)-isopropyl--carbamic acid tert-butyl ester (f) (568 mg, 2.15 mmol, 87 %) as reddish oil.

### c) Preparation of Isopropyl-(2-methylamino-benzyl)-carbamic acid tert-butyl ester

(2-Amino-benzyl)-isopropyl--carbamic acid tert-butyl ester (f) (552 mg, 2.09 mmol) was dissolved into ethyl formate (10 ml). The solution was stirred overnight at 70°C. The solvent was removed in vacuo to give N-formate as red oil. This compound was used in next step without further purification.

Lithium aluminum hydride (79 mg, 2.09 mmol) was suspended in tetrahydrofuran (5 ml). A solution of N-formate in tetrahydrofuran (5 ml) was added gently dropwise to a suspension of lithium aluminum hydride. After stirring for 30 min, ammonium chloride solution was slowly added to quench the reaction. The reaction mixture was filtered and concentrated in vacuo. The crude product was purified by column chromatography (10% ethyl acetate-hexane) to afford Isopropyl-(2-methylamino-benzyl)-carbamic acid tert-butyl ester (g) (126 mg, 0.453 mmol, 22%).

### d)Preparation of [N-methyl-N-2-((tert-butoxycarbonylisopropylamino)methyl) phenyl]carbamic acid 1-chloro-ethyl ester

To a solution of isopropyl-(2-methylamino-benzyl)-carbamic acid tert-butyl ester (119 mg, 0.428 mmol) and N,N-diisopropylethylamine (0.97 ml, 0.556 mmol) in dichloromethane (4 ml) was added chloroethylchloroformate (0.055 ml, 0.514 mmol) at 0°C. The reaction temperature was warm up to room temperature. After stirring for 15 min, the reaction mixture was quenched with water and extracted with dichloromethane. The combined organic phase was washed with water and brine, dried over anhydrous magnesium sulfate, filtered and concentrated in vacuo. The crude product was purified by column chromatography (20% ethyl acetate-hexane) to afford [N-methyl-N-2- ((tert-butoxycarbonylisopropylamino) methyl) phenyl)carbamic acid 1-chloro-ethyl ester as colorless oil (158 mg, 0.409 mmol, 96%). Physical form: colorless oil; EI-MS: m/z 384(M⁺); ¹H-NMR (CDCl₃): δ 0.93∼1.12(6H,m), 1.16∼1.60(12H,m), 3.20(3H,s), 4.02∼4.58(3H,m), 6.49∼6.67(1H,m), 6.98∼7.31(4H, m).

The following compounds in Example 2.1.-2.3. were obtained according to a manner analogous to those of Example 2.

### 2.1.

[ N-2- [(tert-butoxycarbonylisopropylamino) methyl]phenyl]carbamic acid 1-chloro-ethyl ester. Physical form: colorless oil; LC-MS: m/z371(M+1)⁺.

### 2.2.

[ N-2- [(tert-butoxycarbonyl-pentan-3-ylamino) methyl]phenyl] carbamic acid 1-chloro-ethyl ester. Physical form: colorless oil; LC-MS: m/z399(M+1)⁺.

### 2.3.

[N-methyl-N-2-[ (tert-butoxycarbonylmethylamino) methyl]phenyl] carbamic acid 1-chloro-ethyl ester. Physical form: light yellow oil; LC-MS: m/z 357 (M+1)⁺; ¹H-NMR (CDCl₃): δ 1.22∼1.71 (12H, m), 2.64-2.93 (3H, m), 3.21 (3H, s),4.17∼4.58 (2H, m), 6.49-6.63 (1H, m), 7.02∼7.39(4H, m).

### Example 3

### [N-methyl-N-3-((tert-butoxycarbonylmethylamino) acetoxymethyl) pyridin-2-yl] carbamic acid 1-chloro-ethyl ester

### a) Preparation of 2-Chloronicotinoyl chloride

To a suspension of 2-chloronicotinic acid (37.8 g, 0.240 mol) in dry DCM (150 mL) and DMF (0.1 mL) cooled in an ice-bath was added oxalyl chloride (22.9 mL, 0.264 mol) dropwise over a period of 15 min. After stirring for 1 h at 0 °C, the reaction mixture was heated to reflux for 6 h ( the mixture became a clear brownish solution). The solvent and the excess oxalyl chloride was evaporated under reduced pressure. Toluene (100 mL) was added to the residue and the mixture was evaporated. The obtaining residue was purified by vacuum distillation to give 2-chloronicotinoyl chloride (41.7 g, 99%); bp 98-100 °C/2 mmHg, mp 38-39 °C (from Lancaster catalog). Caution: Distillation may need a special care due to the high melting point of the product.

### b) Preparation of t-Butyl 2-chloronicotinate

A solution of 2-chloronicotinoyl chloride (41.7 g, 0.237 mol) in dry THF (400 mL) was cooled in an ice-EtOH-water bath (-5 °C). KOBu^{t} (27.9 g, 0.249 mol) was added portionwise over a period of 30 min and the mixture was stirred for 2 h at 0 °C. THF was evaporated under reduced pressure, and the residue was extracted with EtOAc (600 mL). The EtOAc layer was washed with water and brine, dried over MgSO₄, and evaporated under reduced pressure. The obtaining residue was purified by a short silica gel column chromatography (ca. 100 g of silica gel, eluent: EtOAc/hexane = 1/1) to give t-butyl 2-chloronicotinate (49.0 g, 97%) as an oil; ¹H NMR (CDCl₃): δ 1.64 (s, 9H), 7.32 (dd, J = 4.6 and 7.6 Hz, 1H), 8.06 (dd, J = 2.0 and 7.6 Hz, 1H), 8.48 (dd, J = 2.0 and 4.6 Hz, 1H).

### c) Preparation of t-Butyl 2-(N-methylamino)nicotinate

t-Butyl 2-chloronicotinate(k) (50.0 g, 0.234 mol) was dissolved in a 40% methylamine-methanol solution (300 mL) and the mixture was stirred at room temperature for 30 h. The mixture was evaporated under reduced pressure and the resulting residue was dissolved in EtOAc (750 mL). The EtOAc solution was washed with water, dried over MgSO₄ and evaporated under reduced pressure. The obtaining oily crude product was purified by a short silica gel column chromatography (ca. 300 g of silica gel, eluent: EtOAc/hexane = 1/3) to give t-butyl 2-(N-methylamino)nicotinate(l) (45.0 g, ca. 92%) contaminated by starting meterial; ¹H NMR (CDCl₃): δ 1.57 (s, 9H), 3.05 (d, J = 5.0 Hz, 3H), 6.49 (dd, J = 4.6 and 7.6 Hz, 1H), 7.96 (bs, 1H), 8.04 (dd, J = 2.0 and 7.6 Hz, 1H), 8.28 (dd, J = 2.0 and 4.6 Hz, 1H).

### d) Preparation of 3-Hydroxymethyl-2-(methylamino)pyridine

To a solution of t-butyl 2-(methylamino)nicotinate (45.0 g, 0.216 mol) in dry THF (500 mL) cooled in an ice-bath was added LiAlH₄ (9.84 g, 0.259 mol) portionwise over a period of 30 min. After stiring for 1 h at 0 °C, the mixture was warmed to room temperature and stirred for 2 h. After cooling in an ice-bath, the excess LiAlH₄ was decomposed completely by the careful addition of H₂O (10 mL) and 1N NaOH aqueous solution (10 mL). Na₂SO₄ (100 g) was added and the mixture was filtered through a pad of celite. The filtrate was evaporated under reduced pressure and the resulting residue was purified by column chromatography (400 g of silica gel, eluent: DCM/MeOH = 20/1-10/1) to give the desire product which was further purified by recrystallization from DCM-hexane to give 3-hydroxymethyl-2-(N-methylamino)pyridine(m) (22.7 g, 76%); ¹H NMR (CDCl₃): δ 2.30 (brs, 1H), 3.01 (d, J = 4.6 Hz, 3H), 4.58 (s, 2H), 5.40 (brs, 1H), 6.50 (dd, J = 5.1 and 7.3 Hz, 1H), 7.21 (dd, J = 1.7 and 7.3 Hz, 1H), 8.08 (dd, J = 1.7 and 5.1 Hz, 1H).

### e) Preparation of Methyl 2-aminonicotinate

To a mixture of 2-aminonicotinic acid(n) (30.0g 217 mmol) and 2-chloro1,3-dimethyl imidazolinium chloride(55.2 g 326 mmol) in MeOH (750 ml) was added dropwise triethylamine (91 ml 652 mmol). The resultant mixture was stirred at room temperature for 1 h. The mixture was then evaporated under reduced pressure to afford a residue. The residue was purified by extraction with ethyl acetate (300 ml x 2). The combined organic phase was washed with water (200 ml x 2) and brine (200 ml). Dried over anhydrous sodium sulfate, filtered and concentrated to give an essentially pure methyl 2-aminonicotinate (31.3 g, yield 94%). This compound was used in next step without further purification.

### f) Preparation of Methyl N-formylaminonicotinate

Acetic formic anhydride (AFA) was generated in in the flask by dropwise addition of 98% formic acid(24.5 ml 650 mmol) to acetic anhydride (50.0ml, 530 mmol) maintained at 0°C followed by genntle heating (50°C, 2 h).

The mixture was cooled to room temperature. Methyl 2-aminonicotinate(o) (31.3 g, 206 mmol) was dissolved in dry THF (120 ml) and added to the mixture. The mixture was stirred overnight at room temperature and solvents was removed in vacuo to give an essentially pure methyl N-formylaminonicotinate(p) (37.0 g). This compound was used in next step without further purification.

### g) Preparation of 2-(N-methylamino)-3-hydroxymethylpyridine

To a suspension of lithium aluminium hydride (22.0 g 578 mmol) in dry THF (750 ml) in 3 L flask with condenser, dropping funnel, and mechanical stirrer was added dropwise a solution of N-formylamino-nicotinate (37.0 g, 243 mmol) in 400 ml of dry THF. The mixture was stirred for 30 minutes. To the mixture was added dropwise ethyl acetate (80 ml), MeOH (50 ml), DCM (600 ml), and water (60 ml), and then added anhydroud magnesium sulfate (300 g).

After 1 hour stirring, the mixture was filtered and concentrated in vacuo. The residual solution was crystalized with n-hexane to give pure 2-(N-methylamino)-3-hydroxymethylpyridine (19.8 g, Total yield from 2-aminonicotinic acid was 66 %).

### h) Preparation of (N-methyl-N-3-((tert-butoxycarbonylmethylamino)acetoxymethyl) pyridin-2-yl] carbamic acid 1-chloro-ethyl ester

2-(N-methylamino)-3-hydroxymethylpyridine (22 g,0.159 mol) and diisopropylamine (36.1 mL, 0.207 mol, 1.3 eq.) were dissolved in dichloromethane(1L) and cooled in ethanol-ice bath(ca-13°C). 1-Chloroethyl chloroformate(17.5 mL, 0.161 mol, 1.01 eq.) was added dropwise over a period of 1h and the mixture was stirred for 1h.Boc-sarcosine(39.2 g, 0.207 mol, 1.3 eq.) was added to the stirring mixture and WSC(39.7 g, 0.207 mol, 1.3 eq.) was added portionwise over a period of 10 min. To the mixture was added DMAP(5.8 g, 0.047 mol, 0.3 eq.) and the mixture was stirred for 2h at-7°C. The reaction mixture was concentrated at 25°C and the residue was dissolved in diethylether(1L). The solution was transferred to the separate funnel and washed with 0.1N-HCl(500 mL x 3), water (500 mL), NaHCO₃ aq.(500 mL) and brine(500 mL x 2) successively, dried over MgSO₄ and concentrated under reduced pressure. The obtained residue(48.2 g, ca 72.9% yield) was used for the next step without purification. ¹H-NMR (270 MHz,CDCl₃): δ 1.42 (9H, d, J = 24.1), 1.57 (1.5H, br.s), 1.88 (1.5H, br.s), 2.94 (3H, s), 3.37 (3H, s), 4.00 (2H, d, J = 21.1), 5.18 (2H, d, J = 13.5), 6.58 (1H, q, j = 5.45, 11.0), 7.30 (1H, s), 7.82 (1H, d, J = 6.9), 8.47 (1H, s); FAB-MS: m/z 416 (M+H)⁺.

The following compounds in Example 3.1.-3.23. were obtained according to a manner analogous to those of Example 3.

### 3.1.

[N-methyl-N-2-[(tert-butoxycarbonylmethylamino)acetoxymethyl]phenyl]carbamic acid chloromethylester. Physical form: colorless oil; LC-MS: m/z401(M+1)⁺; ¹H-NMR (CDCl₃): δ1.37 (s, 9/2H), 1.46 (s, 9/2H), 2.93 (s, 3H), 3.29 (s, 3H), 3.93-4.02 (m, 2H), 3.95 (s, 1H), 4.03 (s, 1H), 5.15 (s, 2H), 5.57-5.84 (m, 2H), 7.15-7.49 (m, 4H).

### 3.2.

[N-methyl-N-2-[ (tert-butoxycarbonylmethylamino)acetoxymethyl] phenyl]carbamic acid 1-chloro-ethyl ester. Physical form: colorless oil; LC-MS: m/z415(M+1)⁺; ¹H-NMR (CDCl₃): δ1.37 (s, 9/2H), 1.46 (s, 9/2H), 1.55-1.60 (m, 9/4H), 1.90 (d, d = 5.6 Hz, 3/4H), 2.93 (s, 3H), 3.29 (s, 3H), 3.94-4.05 (m, 2H), 5.10-5.19 (m, 2H), 6.51-6.63 (m. 1H), 7.11-7.48 (m, 4H).

### 3.3.

[N-methyl-N-2-[(tert-butoxycarbonylmethylamino)acetoxymethyl]-4,5-difluoro-phenyl] carbamic acid 1-chloro-ethyl ester. Physical form: colorless oil; LC-MS: m/z451(M+1)⁺; ¹H-NMR (CDCl₃): δ1.39 (s, 9/2H), 1.47 (s, 9/2H), 1.59-1.64 (m, 9/4H), 1.89 (d, J = 5.3 Hz, 3/4H), 2.94 (s, 3H), 3.26 (s, 3H), 3.96-4.03 (m, 2H), 5.07-5.16 (m, 2H), 6.51-6.61 (m, 1H), 6.96-7.37 (m, 2H).

### 3.4.

[N-methyl-N-2-[(tert-butoxycarbonylmethylamino)acetoxymethyl]-4-fluoro-phenyl] carbamic acid 1-chloro-ethyl ester. Physical form: light yellow oil; LC-MS: m/z 433 (M+1)⁺; ¹H-NMR (CDCl₃): δ1.38 (9/2H, s), 1.44 (9/2H, s), 1.55 (3/2H, d, J=5.61Hz ), 1.59 (3/2H, d, J=5.61Hz ), 2.92 (3H, brs), 3.18-3.30 (3H, m), 3.87∼4.08 (2H, m), 4.95∼5.18 (2H, m), 6.44-6.60 (1H, m), 6.90-7.21 (3H, m).

### 3.5.

[N-methyl-N-2-[(tert-butoxycarbonylmethylamino)acetoxymethyl]-4,5-dimethoxyphenyl]carbamic acid chloromethyl ester. Physical form: colorless oil; LC-MS: m/z 461 (M+1)⁺; ¹H-NMR(CDCl₃): δ1.35-1.45(9H,m), 2.92(3H,s), 3.27(3H,s), 3.80-4.00(2H,m),3.86 (3H,s), 3.90(3H,s), 5.05(2H,m), 5.62-5.84(2H,m), 6.62(1H,br.s), 6.93( 1 H,br.s).

### 3.6.

[N-methyl-N-2-[(tert-butoxycarbonylmethylamino)acetoxymethyl]-5-fluorophenyl]carbamic acid 1-chloro-ethyl ester. Physical form: colorless oil; LC-MS: m/z 433 (M+1)⁺; ¹H-NMR(CDCl₃): δ1.37 (s, 9/2H), 1.46 (s, 9/2H), 1.56-1.62 (m, 9/4H), 1.89 (d, J = 5.3 Hz, 3/4H), 2.92 (s, 3H), 3.28 (s, 3H), 3.93-4.02 (m, 2H), 5.05-5.13 (m, 2H), 6.52-6.60 (m, 1H), 6.83-7.09 (m, 2H), 7.41-7.49 (m, 1H).

### 3.7.

[N-methyl-N-2-[(tert-butoxycarbonylmethylamino)acetoxymethyl]-6-methyl-phenyl] carbamic acid 1-chloro-ethyl ester. Physical form: colorless oil; LC-MS: m/z 429 (M+1)⁺; ¹H-NMR(CDCl₃): 1.36-1.49(9H, m), 1.61(3H, s), 2.18-2.25(3H, m), 2.92(3H, s), 3.21(3H, s), 3.91-4.05(2H, m), 5.04-5.22(2H. m), 6.51-6.64(1H, m).

### 3.8.

[N-methyl-N-2-[(tert-butoxycarbonylmethylamino)acetoxymethyl)-4-chloro-phenyl] carbamic acid 1-chloro-ethyl ester. Physical form: colorless oil; LC-MS: m/z 449 (M+ 1)⁺; ¹H-NMR(CDCl₃): δ 1.38-1.47(9H, m), 1.59(3H, s), 2.94(3H, s), 3.27(3H, s), 3.94-4.08(2H, m), 5.05-5.17(2H, m), 6.55(1H, m), 7.02-7.21(1H, m), 7.36(1H, m), 7.45(1H, s).

### 3.9.

[N-(tert-butoxycarbonylmethylamino)acetoxyethyl-N-2,4-difluoro-phenyl]carbamic acid 1-chloro-ethyl ester. Physical form: colorless oil; LC-MS: m/z 451 (M+1)⁺; ¹H-NMR (CDCl₃): δ 1.41 (s, 9/2H), 1.46 (s, 9/2H), 1.59-1.64 (m, 9/4H), 1.89 (d, d = 5.6 Hz, 3/4H), 2.89 (s, 3H), 3.85-4.00 (m, 4H), 4.27-4.36 (m, 2H), 6.49-6.83 (m, 3H), 7.19-7.32 (m. 1H).

### 3.10.

[N-methyl-N-2-[(tert-butoxycarbonylmethylamino)acetoxymethyl]-5-chloro-phenyl] carbamic acid 1-chloro-ethyl ester. Physical form: colorless oil; LC-MS: m/z 449 (M+1)⁺; ¹H-NMR (270 MHz,CDCl₃): δ 1.37 (4.5H, s), 1.46 (4.5H, s), 1.59 (2H, br.s), 1.89 (1H, br.s), 2.92 (3H, s), 3.28 (3H, s), 3.94 (1H, s), 4.02 (1H, s), 5.06 (1H, br.s), 5.10 ( 1 H, br.s), 6.39-6.59 (1H, m), 7.17-7.23 (1H, m), 7.31-7.43 (2H, m)

### 3.11.

[N-methyl-N-2-[(tert-butoxycarbonylmethylamino)acetoxymethyl]-5-nitro-phenyl] carbamic acid 1-chloro-ethyl ester. Physical form: yellow oil; LC-MS: 460(M+1)⁺; ¹H-NMR (CDCl₃): δ1.22∼1.70(11H, m), 1.88(1H,d, J=4.95Hz), 2.90(3H, br.s), 3.29(3H, br.s). 3.90-4.08 (2H, m), 5.03∼5.28(2H, m), 6.46∼6.61(1H, m), 7.55∼7.72(1H, m), 7.96-8.27(2H,m).

### 3.13.

[N-methyl-N-2-[(tert-butoxycarbonylmethylamino)acetoxymethyl]-3-fluoro-phenyl] carbamic acid 1-chloro-ethyl ester. Physical form: colorless oil; LC-MS: m/z 433 (M+1)⁺; ¹H-NMR (CDCl₃): δ 1.38 (s, 9/2H), 1.44 (s, 9/2H), 1.58-1.62 (m, 9/4H), 1.89 (d, J = 5.6 Hz, 3/4H), 2.91 (s, 3H), 3.28 (s, 3H), 3.90-3.98 (m, 2H), 5.00-5.35 (m, 2H), 6.50-6.61 (m, 1H), 6.96-7.14 (m, 2H), 7.31-7.42 (m, 1H).

### 3.14.

[N-methyl-N-2-[(tert-butoxycarbonylmethylamino)acetoxymethyl]-5-cyano-phenyl] carbamic acid 1-chloro-ethyl ester. Physical form: colorless oil; LC-MS: m/z 440 (M+1)⁺; ¹H-NMR(CDCl₃): δ 1.36-1.48(9H, m), 1.57(3H, s), 2.93(3H, d, J=4.9Hz), 3.29(3H, s), 3.88-4.04(2H, m), 5.06-5.20(2H, m), 6.53(1H, m), 6.81-6.95(1H, m), 7.46-7.68(2H, m).

### 3.15.

[N-methyl-N-2-[(tert-butoxycarbonylmethylamino)acetoxymethyl]-3-chloro-phenyl] carbamic acid 1-chloro-ethyl ester, Physical form: colorless oil; LC-MS: m/z 449 (M+1)⁺; ¹H-NMR(CDCl₃): δ 1.39 (s, 9/2H), 1.44 (s, 9/2H), 1.57-1.61 (m, 9/4H), 1.89 (m, 3/4H), 2.91 (s, 3H), 3.26 (s, 3H), 3.93-4.03 (m, 2H), 5.06-5.39 (m, 2H), 6.50-6.56 (m, 1H), 7.07-7.45 (m, 3H).

### 3.16.

[N-methyl-N-2-[(tert-butoxycarbonylmethylamino)acetoxymethyl]-4-cyano-phenyl] carbamic acid 1-chloro-ethyl ester. Physical form: colorless oil; LC-MS: m/z 440 (M+1)⁺.

### 3.17.

[N-methyl-N-2-[(tert-butoxycarbonylmethylamino)acetoxymethyl]-5-trifluoromethylphenyl]carbamic acid 1-chloro-ethyl ester. Physical form: colorless oil; LC-MS: m/z 483 (M+1)⁺; ¹H-NMR(CDCl₃): δ 1.36-1.45(9H, m), 1.56(3H, s), 2.93(3H, s), 3.31(3H, s), 3.95-4.08(2H, m), 5.12-5.21(2H, m), 6.56(1H, m), 7.37-7.60(3H, m).

### 3.18.

[N-methyl-N-2-[(tert-butoxycarbonylamino)acetoxymethyl]-3-chloro-phenyl]carbamic acid 1-chloro-ethyl ester. Physical form: colorless oil; LC-MS: m/z 449 (M+1)⁺; ¹H-NMR(CDCl₃): δ 1.44 (s, 9H), 1.57-1.61 (m, 2H), 1.85-1.89 (m, 1H), 3.24 (s, 1H), 3.26 (s, 2H), 3.88-3.94 (m, 2H), 5.03 (bs, 1H), 5.13-5.36 (m, 2H), 6.48-6.56 (m, 1H), 7.08-7.45 (m, 3H).

### 3.19.

[N-ethyl-N-2-[(tert-butoxycarbonylmethylamino)acetoxymethyl]-3-chloro-phenyl] carbamic acid 1-chloro-ethyl ester. Physical form: colorless oil; LC-MS: m/z 463 (M+1)⁺; ¹H-NMR(CDCl₃): δ 1.14-1.21 (m, 3H), 1.41 (s. 9/2H), 1.44 (s, 9/2H), 1.56-1.66 (m, 9/4H), 1.89 (m, 3/4H). 2.91 (s, 3H), 3.39-3.52 (m, 1H). 3.80-4.05 (m, 3H), 5.03-5.14 (m, 1H), 5.26-5.40 (m, 1H), 6.49-6.60 (m, 1H), 7.04-7.46 (m, 3H).

### 3.20.

[N-methyl-N-3-[(tert-butoxycarbonylamino)acetoxymethyl]pyridin-2-yl]carbamic acid 1-chloro-ethyl ester. Physical form: colorless oil; LC-MS: m/z 401 (M)⁺; ¹H-NMR (270 MHz, CDCl₃): δ 1.43 (9H, s), 1.55 (3/2H, br.s), 1.87 (3/2H, br.s), 3.20 (3H, s), 3.93 (1H, s), 3.96 (1H, s), 5.15 (1H, br.s), 5.20 ( 1 H, br.s), 5.64 ( 1 H, br.s), 6.57 (1H, m), 7.32 ( 1 H, m), 7.85 (1H, m), 8.46 (1H, m)

### 3.21.

[N-methyl-N-2-[(tert-butoxycarbonylmethylamino)acetoxymethyl]-3-methyl-pheny] carbamic acid 1-chloro-ethyl ester. Physical form: colorless oil; LC-MS: m/z 429(M+1)⁺.

### 3.22.

[N-ethoxycarbonyl-N-2-((tert-butoxycarbonylmethylamino)acetoxymethyl)pheny]carbamic acid 1-chloro-ethyl ester. Physical form: colorless oil; LC-MS: m/z 473(M+1)⁺; ¹H-NMR (CDCl₃): δ 1.24(3H, t, J=6.9Hz) 1.41-1.47(9H, m), 1.66(3H, d, J=5.6Hz), 2.91(3H, d, J=3.6Hz), 3.92-4.00(2H, m), 4.26(2H, q, J=6.9Hz), 5.13(2H, m), 6.53(1H, q, J=5.6Hz), 7.12-7.21(1H, m), 7.38-7.50(3H, m).

### 3.23.

[N-pivaloyl-N-2-((tert-butoxycarbonylmethylamino)acetoxymethyl)phenyl]carbamic acid 1-chloro-ethyl ester. Physical form: colorless oil; LC-MS: m/z 485(M+1)⁺; ¹H-NMR (CDCl₃): δ 1.33-1.49(18H, m), 1.65(3H, d, J=5.6Hz), 2.91(3H, d, J=4.0Hz), 3.91-4.05(2H, m), 5.03-5.26(2H, m), 6.50(1H, q, J=5.6Hz), 7.06-7.19(1H, m), 7.36-7.53(3H, m).

### Example 5

### [[N-methyl-N-2-(acetoxymethyl)phenyl]carbamoyloxy]methyl-1-[(2R,3R)-2- (2,5-difluorophenyl)-2-hydroxy-3-[4-(4-cyanophenyl)thiazol-2-yl]butyl]-1H-[1,2,4]triazol-4-ium chloride

### a) Preparation of [[N-methyl-N-2-(acetoxymethyl)phenyl]carbamoyloxy] methyl-1-[(2R,3R)-2-(2,5-difluorophenyl)-2-hydroxy-3-[4-(4-cyanophenyl)thiazol-2-yl]butyl]-1H-[1,2,4]triazol-4-ium chloride

A solution of 1-[3-[4-(4-cyano-phenyl)-thiazol-2-yl]-2-(2,5-difluoro-phenyl)-2-hydroxy-butyl-1H-[1,2,4]triazol (200 mg, 0.457 mmol), sodium iodide (6.8 mg 0.045 mmol) and acetic acid 2-(chloromethoxycarbonyl-methyl-amino)-benzyl ester 150 mg, 0.552 mmol) was stirred for 6h at ambient temperature and then at 80 °C for 3h under Ar atmosphere. The reaction mixture was concentrated under reduced pressure and the resulting material was eluted on a column of silica gel (Kusano Si-5, eluent dichloromethane:methanol = 20:1). The fractions containing the product were concentrated under reduced pressure giving the title compound a) as colorless amorphous (204.5 mg, 63 %). ¹H-NMR (270 MHz, DMSO-d₆): δ 1.20 (3H, d, J = 6.9), 1.99 (3H, s), 3.12 (2.4H, s), 3.15 (0.6H, s), 4.15 (1H, q, J = 7.3), 4.79-4.91 (3H, m), 5.09 (1H, d, J = 14.8), 5.76 (1H, s), 5.90-6.10 (1.6H, m), 6.17 (0.4H, br.s), 6.61-6.66 (1H, m), 7.05-7.15 (1H, m), 7.26-7.44 (6H, m), 7.91-7.96 (2H, m), 8.20-8.24 (2H, m), 8.49 (1H, s), 9.01 (0.8H, br.d, J = 3.6), 9.12 (0.2H, br.s), 10.16 (0.8H, br.d, J = 4.9), 10.27 (0.2H, br.s); FAB-MS: 673 (M-Cl)⁺; Ratio of Retention Time in HPLC: 1.79 (see Example D).

The following compounds in Example 5.1.-5.7. were obtained according to a manner analogous to those of Example 5.

### 5.1.

[[N-methyl-N-phenyl]carbamoyloxy]methyl-1-[(2R,3R)-2-(2,5-difluorophenyl)-2-hydroxy-3-[4-(4-cyanophenyl)thiazol-2-yl]butyl]-1H-[1,2,4]triazol-4-ium chloride. Physical form: colorless amorphous powder; FAB-MS: 601 (M-Cl) ⁺; Ratio of Retention Time in HPLC: 1.10(see Example D).

### 5.2.

1-[[N-methyl-N-3-(acetoxymethyl)pyridin-2-yl]carbamoyloxy]ethyl-1-[(2R,3R)-2-(2,5-difluorophenyl)-2-hydroxy-3- [4-(4-cyanophenyl)thiazol-2-yl]butyl]-1H-[1,2,4]triazol-4-ium chloride hydrochloride. Physical form: colorless amorphous powder; FAB-MS: 688 (M-Cl) ⁺; Ratio of Retention Time in HPLC: 0.82(see Example D). ¹H-NMR(DMSO-d6): δ 1.20(3H,d,J=7.3Hz), 1.50-1.88(3H,m), 2.02(3H,m), 3.18(3H.br.s), 4.16(1H,m), 4.70-5.12(4H,m), 6.80(1H,m), 7.05-7.48(4H,m), 7.91(3H,br.d,J=8.3Hz), 8.21(2H,d,J=8.3Hz), 8.46(2H,br.s), 9.21(1H,m), 10.4(1H,m).

### 5.7.

[[N-2-(methyl)phenyl-N-2-(acetoxy)ethyl]carbamoyloxy]methyl-1-[(2R,3R)-2-(2,5-difluorophenyl)-2-hydroxy-3-[4-(4-cyanophenyl)thiazol-2-yl]butyl]-1H-[1,2,4]triazol-4-ium iodide. Physical form: colorless amorphous powder; FAB-MS: 687 (M-I)⁺; Ratio of Retention Time in HPLC:1.79(see Example D).

### Example 6

### 1-[[N-methyl-N-2-(isopropylaminomethyl)phenyl] carbamoyloxy] ethyl-1-[(2R,3R)-2-(2,5-difluorophenyl)-2-hydroxy-3-[4-(4-cyanophenyl)thiazol-2-yl]butyl]-1H-[1,2,4]triazol-4-ium chloride hydrochloride

### a) Preparation of 1-[[N-methyl-N-2-(t-butoxycarbonyl-isopropylaminomethyl)phenyl]carbamoyloxy]ethyl-1-[(2R,3R)-2-(2,5-difluorophenyl)-2-hydroxy-3- 4-(4-cyanophenyl)thiazol-2-yl]butyl]-1H-[1,2,4]triazol-4-ium chloride

To a solution of [N-methyl-N-2- ((tert-butoxycarbonylisopropylamino) methyl) phenyl] carbamic acid 1-chloro-ethyl ester (143 mg, 0.342 mmol) in acetonitrile (1 ml) was added the azole compound (163 mg, 0.372 mmol) and catalytic amount of sodium iodide at 70°C. After stirring overnight, the solvent was removed and extracted with ethyl acetate. The organic phase was washed with water and brine. The solvent was removed in vacuo. The residue was purified by column chromatography (ethyl acetate to 10% methanel-dichloromethane) to afford 1-[[N-methyl-N-2-(t-butoxycarbonyl-isopropylaminomethyl) phenyl] carbamoyloxy]ethyl-1-[(2R,3R)-2-(2,5-difluorophenyl)-2-hydroxy-3-[4-(4-cyanophenyl) thiazol-2-yl]butyl]-1H-[1,2,4]triazol-4-ium chloride (155 mg, 0.189 mmol, 51%) as off-white amorphous.

### b) Preparation of 1-[[N-methyl-N-2-(isopropylaminomethyl)phenyl]carbamoyloxy]ethyl-1-[(2R,3R)-2-(2,5-difluorophenyl)-2-hydroxy-3-[4-(4-cyanophenyl)thiazol-2-yl]butyl]-1H-[1,2,4]triazol-4-ium chloride hydrochloride

To a solution (1 ml) of 1-([N-methyl-N-2-(t-butoxycarbonylisopropylamino-methyl) phenyl] carbamoyloxy]ethyl-1-[(2R,3R)-2-(2,5-difluorophenyl)-2-hydroxy-3-[4-(4-cyanophenyl)thiazol-2-yl]butyl]-1H-[1,2,4] triazol-4-ium chloride (148 mg, 0.180 mmol) in ethyl acetate (1 ml) was added 4N hydrogen chloride ethyl acetate solution (1 ml) at room temperature. After stirring for 2 hours, the precipitate was filtered and washed with ethyl acetate. The precipitate was dried up to affordl-[[N-methyl-N-2-(isopropylaminomethyl)phenyl]carbamoyloxy]ethyl-1-[(2R,3R)-2-(2,5-difluoraphenyl)-2-hydroxy-3-[4(4-cyanophenyl)thiazol-2-yl]butyl]-1H[1,2,4]triazol-4-ium chloride hydrochloride (137 mg, 0.180 mmol, quant) as off-white amorphous. Physical form: off-white amorphous powder; FAB-MS: 686 (M-HCl-Cl) ⁺; ¹H-NMR (DMSO): δ 1.10∼1.64 (12H, m), 3.10∼3.30 (3H, m), 3.79∼4.28 (2H, m), 4.56∼5.22 (5H, m), 6.59∼6.84 (1H, m), 7.02∼7.49 (6H, m), 7.99 (2H, d, J=8.25Hz), 8.20 (2H, d, J=7.92Hz), 8.48 (1H, s), 9.08∼9.39 (3H, m), 10.35-10.62 (1H, m).

The following compounds in Example 6.1.-6.3. were obtained according to a manner analogous to those of Example 6.

### 6.1.

1-[[N-2- [(isopropylamino) methyl]phenyl]carbamoyloxy]ethyl-1-[(2R,3R)-2-(2,5-difluorophenyl)-2-hydroxy-3-[4-(4-cyanophenyl)thiazol-2-yl]butyl]-1H-[1,2,4]triazol-4-ium chloride hydrochloride. Physical form: colorless amorphous powder; FAB-MS: 672 (M-HCl-Cl) ⁺. ¹H-NMR (DMSO): δ 1.09∼1.39 (6H, m), 1.82 (3H, brs), 3.30 (1H, brs), 3.98-4.20 (3H, m), 4.78 (1H, dd, J=4.95, 9.51Hz), 5.08 (2H, d, J=14.18Hz), 6.73∼6.92 (1H, m), 7.05∼7.48 (7H, m), 7.58∼7.68 (1H, m), 7.92 (2H, d, J= 7.92Hz), 8.22 (2H, d, J=8.25Hz), 8.49 (1H, d, J=2.97Hz), 9.17-9.37 (3H, m), 9.96 (1H, brs), 10.50 (1H, d, J=13.86Hz).

### 6.2.

1-[[N-2-[ (pentan-3-ylamino) methyl]phenyl]carbamoyloxy]ethyl-1-[(2R,3R)-2-(2,5-difluorophenyl)-2-hydroxy-3-[4-(4-cyanophenyl)thiazol-2-yl]butyl]-1H-[1,2,4]triazol-4-ium chloride hydrochloride. Physical form: colorless amorphous powder; FAB-MS: 700 (M-HCl-Cl)⁺. ¹H-NMR (DMSO): δ 0.78∼0.97 (6H, m), 1.07∼1.28 (3H, m), 1.59∼1.90 (4H, m), 2.96 (1H, brs), 4.08∼4.19 (3H, m), 4.79 (1H, dd, J=5.28, 9.51Hz), 5.08 (2H, d, J=14.18Hz), 6.74∼6.99 (2H, m), 7.08∼7.46 (6H, m), 7.92 (1H, dd, J=1.32, 3.25Hz), 8.19 (2H, d, J= 8.58Hz), 8.48 (2H, d, J=2.97Hz), 9.21 (1H, brs), 9.30 (1H, s), 10.00 (1H, brs), 10.54 (1H, d, J=16.82Hz).

### 6.3.

1-[[N-methyl-N-2- [(methylamino) methyl] phenyl]carbamoyloxy]ethyl-1-[(2R,3R)-2-(2,5-difluorophenyl)-2-hydroxy-3-[4-(4-cyanophenyl)thiazol-2-yl]butyl]-1H-[ 1,2,4]triazol-4-ium chloride hydrochloride. Physical form: colorless amorphous powder; FAB-MS: 658 (M-HCl-Cl)⁺. ¹H-NMR (DMSO): δ 1.13∼1.32 (4H, m), 1.48∼1.65 (2H, m), 1.78∼1.96 (1H, m), 3.08∼3.32 (4H, m), 3.80∼4.25 (3H, m), 4.70∼4.89 (1H, m), 5.06 (1H, d, J=13.85Hz), 6.38 (1H, brs), 6.58∼6.84 (1H, m), 7.05∼7.52 (7H, m), 7.72∼8.15 (1H, m), 7.94 (2H, d, J=8.24Hz), 8.22 (2H, d, J=8.25Hz), 8.49 (1H, s), 9.08∼9.42 (1H, m), 9.78 (1H, brs), 10.30∼10.77 (1H, m).

### Example 7

### 1-[[N-methyl-N-3-[(methylamino)acetoxymethyl] pyridin-2-yl]carbamoyloxy]ethyl-1-[(2R,3R)-2-(2,5-difluorophenyl)-2-hydroxy-3-[4-(4-cyanophenyl)thiazol-2-yl]butyl]-1H-[1,2,4]triazol-4-ium chloride hydrochloride

### a) Preparation of 1-[[N-methyl-N-3-[(t-butoxycarbonylmethylamino) acetoxymethyl] pyridin-2-yl]carbamoyloxy]ethyl-1-[(2R,3R)-2-(2,5-difluorophenyl)-2-hydroxy-3-[4-(4-cyanophenyl)thiazol-2-yl]butyl]-1H-[1,2,4]triazol-4-ium chloride

[N-methyl-N-3-((tert-butoxycarbonylmethylamino)acetoxymethyl)pyridin-2-yl]carbamic acid 1-chloro-ethyl ester(q) (55 g, 0.132 mol, 1.4 eq) and the azole compound of Example 5a) (41.2 g, 0.0944 mol) was dissolved in CH₃CN(350 mL) and warmed to 45-50°C. To the solution was added NaI(19.7 g, 0.131 mol, 1.4 eq) and stirred for 15 h. After cooling to room temperature, the reaction mixture was concentrated under reduced pressure. The obtained residue was purified by silicagel columnchromatography((eluent: fromAcOEt to AcOEt/MeOH (10/1,v/v) gradient) to give the product as its iodide form(78.7 g, 88.4% yield).

The iodide (66.5 g, 0.07 mol) was dissolved in MeOH(300 mL) and distilled water(200 mL) at 0°C and strong anion exchange resin[Dia Ion SA10A(200 g)] was added to the solution. The mixture was stirred using an evaporator. After 1 h, the mixture was filtered, washed with methanol and the filtrate was evaporated. The obtained residue was diluted with water(200 mL), brine(200 mL) and ethyl acetate(500 mL). The organic layer was extracted with ethyl acetate and combined ethyl acetate layer was dried over Na₂SO₄ and concentrated under reduced pressure. The obtained residue was purified by silicagel column chromatography(( eluent:DCM/MeOH(10/1, v/v)) to give the product (52.1 g, 86.7% yield).

### b) Preparation of 1-[[N-methyl-N-3-[(methylamino)acetoxymethyl]pyridin-2-yl] carbamoyloxy]ethyl-1-[(2R,3R)-2-(2,5-difluorophenyl)-2-hydroxy-3-[4-(4-cyanophenyl)thiazol-2-yl]butyl]-1H-[1,2,4]triazol-4-ium chloride hydrochloride

1-[[N-methyl-N-3-[(t-butoxycarbonylmethylamino)acetoxymethyl]pyridin-yl]carbamoyloxy]ethyl-1-[(2R,3R)-2-(2,5-difluorophenyl)-2-hydroxy-3-[4-(4-cyanophenyl)thiazol-2-yl]butyl]-1H-[1,2,4]triazol-4-ium chloride (51.5 g, 0.06 mol) was dissolved in dry ethyl acetate(900 mL) and cooled to 0°C. To this solution 4N-HCl/EtOAc(0.8 mol, 200 mL) was added dropwise. The mixture was stirred vigorously for 4 h at room temperature. After filtration, the filtrate was washed with EtOAc under N₂. The obtained white solid was dried through N₂ for 2 d, further dried at 70°C under reduced pressure for 24 h. The dried solid was dissolved in distilled water (2 L) and washed with dichloromethane (2 L x 5) and hexane( 1 L x 2) and water layer was freezed dried to give the final product (32.8 g). ¹H-NMR (400 MHz, 100°C, DMSO-d₆): δ 1.25 (3H, d, J = 6.1), 1.72 (3H, br.s), 2.58 (3H, d, J = 4.0), 3.21 (3H, s), 3.94 (2H, d, J = 2.8), 4.16 (1H, q, J = 6.1), 4.85-4.90 (1H, m), 5.08-5.14 (1H+2H, m), 6.84 (1H, q, J = 6.0), 7.14-7.17 (2H, m), 7.18-7.27 (1H. m), 7.41-7.45 (1H, m), 7.86 (2H, d, J = 8.4), 8.00 (1H, d, J = 6.8), 8.16 (2H, d, J = 8.4), 8.28 (1H, s), 8.44-8.48 (1H, m), 9.17 (1H, s), 10.47(1H, d, J = 18.0); FAB-MS: m/z 717 (M-2HCl-Cl)⁺:

The following compounds in Example 7.1.-7.23. were obtained according to a manner analogous to those of Example 7.

### 7.1.

[[N-methyl-N-2-[(methylamino)acetoxymethyl]phenyl]carbamoyloxy]methyl-1[(2R,3R)-2-(2,5-difluorophenyl)-2-hydroxy-3-[4-(4-cyanophenyl)thiazol-2-yl]butyl]-[1,2,4]triazol-4-ium chloride hydrochloride. Physical form: colorless amorphous powder; FAB-MS: m/z 702 (M-HCl-Cl)⁺; Ratio of Retention Time in HPLC: 0.78(see Example D).

### 7.2.

1-[[N-methyl-N-2-[(methylamino)acetoxymethyl]phenyl] carbamoyloxy]ethyl-1[(2R,3R)-2-(2,5-difluorophenyl)-2-hydroxy-3-[4-(4-cyanophenyl)thiazol-2-yl]butyl]-1H-[1,2,4] triazol-4-ium chloride hydrochloride. Physical form: colorless amorphous powder; FAB-MS: m/z 716 (M-HCl-Cl) ⁺; Ratio of Retention Time in HPLC: 0.75 (see Example D).

### 7.3.

1-[[N-methyl-N-2-(methylamino)acetoxymethyl-4,5-difluorophenyl]carbamoyloxy]ethyl-1-[(2R,3R)-2-(2,5-difluorophenyl)-2-hydroxy-3-[4-(4-cyanophenyl)thiazol-2-yl]butyl]-1H-[1,2,4]triazol-4-ium chloride hydrochloride. Physical form: colorless amorphous powder; FAB-MS: m/z 752 (M-HCl-Cl) ⁺; Ratio of Retention Time in HPLC: 0.94(see Example D).

### 7.4.

1-[[N-methyl-N-2- (methylamino)acetoxymethyl-4-fluoro-phenyl]carbamoyloxy]ethyl-1-[(2R,3R)-2-(2,5-difluorophenyl)-2-hydroxy-3-[4-(4-cyanophenyl)thiazol-2-yl]butyl]-1H-[1,2,4]triazol-4-ium iodide hydrochloride. Physical form: colorless amorphous powder; FAB-MS: m/z 734 (M-HCl-I)⁺; Ratio of Retention Time in HPLC: 0.83(see Example D). ¹H-NMR (DMSO): δ 1.15∼1.30 (3H, m), 1.49∼1.61 (3H, m), 1.79∼1.89 (1H, m), 2.52∼2.65 (3H, m), 3.05∼3.21 (4H, m), 3.98∼4.22 (2H,m), 3.67∼5.25 (5H, m), 6.66∼6.93 (1H, m), 7.03∼7.53 (4H, m), 7.95 (2H, d, J=8.24Hz), 8.21 (2H, d, J=8.25Hz), 8.48 (1H, brs), 9.06∼9.30 (1H, m), 9.32∼9.62 (2H, m), 10.32∼10.53 (1H, m).

### 7.5.

[[N-methyl-N-2-(methylamino)acetoxymethyl-4,5-dimethoxy-phenyl]carbamoyloxy] methyl-1-[(2R,3R)-2-(2,5-difluorophenyl)-2-hydroxy-3-[4-(4-cyanophenyl)thiazol-2-yl]butyl]-1H-[1,2,4]triazol-4-ium iodide hydrochloride. Physical form: colorless amorphous powder; FAB-MS: m/z 762 (M-HCl-I)⁺; Ratio of Retention Time in HPLC: 0.79(see Example D). ¹H-NMR(DMSO-d6): δ 1.18(3H,d,J=7.3Hz), 2.50(3H,br.s), 3.12(3H,br.s), 3.65∼4.18(11H,m), 4.79∼5.12(2H,m), 5.90∼7.38(7H,m), 7.92(2H,br.d,J=8.2Hz), 8.21(2H,br.d,J=8.2Hz), 8.50(1H,br.s), 9.05(1H,br.s), 9.45(2H,br.s), 10.2(1H,br.s).

### 7.6.

1-[[N-methyl-N-2-(methylamino)acetoxymethyl-5-fluoro-phenyl]carbamoyloxy]ethyl-1-[(2R,3R)-2-(2,5-difluorophenyl)-2-hydroxy-3-[4-(4-cyanophenyl)thiazol-2-yl]butyl]-1H-[1,2,4]triazol-4-ium chloride hydrochloride. Physical form: colorless amorphous powder; FAB-MS: m/z 734 (M-HCl-Cl)⁺; Ratio of Retention Time in HPLC: 0.80(see Example D).

### 7.7.

1-[[N-methyl-N-2-(methylamino)acetoxymethyl-6-methyl-phenyl]carbamoyloxy]ethyl-1-[(2R,3R)-2-(2,5-difluorophenyl)-2-hydroxy-3-[4-(4-cyanophenyl)thiazol-2-yl]butyl]-1H-[1,2,4]triazol-4-ium iodide hydrochloride. Physical form: pale yellow solid. LC-MS: m/z 731(M+H)⁺. Ratio of Retention Time in HPLC: 0.70(see Example D).

### 7.8.

1-[[N-methyl-N-2-(methylamino)acetoxymethyl-4-chloro-phenyl]carbamoyloxy] ethyl-1-[(2R,3R)-2-(2,5-difluorophenyl)-2-hydroxy-3-[4-(4-cyanophenyl)thiazol-2-yl]butyl]-1H-[1,2,4]triazol-4-ium iodide hydrochloride. Physical form: pale yellow solid. LC-MS: m/z 750 (M-HCl-I)⁺; Ratio of Retention Time in HPLC: 0.70(see Example D).

### 7.9.

1-[[N-(methylamino)acetoxyethyl-N-2,4-difluorophenyl]carbamoyloxy]ethyl-1-[(2R,3R)-2-(2,5-difluorophenyl)-2-hydroxy-3-[4-(4-cyanophenyl)thiazol-2-yl]butyl]-1H-[1,2,4] triazol-4-ium chloride hydrochloride. Physical form: colorless amorphous powder; FAB-MS: m/z 752 (M-HCl-Cl)⁺; Ratio of Retention Time in HPLC: 0.76(see Example D).

### 7.10.

1-[[N-methyl-N-2-(methylamino)acetoxymethyl-5-chloro-phenyl]carbamoyloxy]ethyl-1-[(2R,3R)-2-(2,5-difluorophenyl)-2-hydroxy-3-[4-(4-cyanophenyl)thiazol-2-yl]butyl]-1H-[1,2,4]triazol-4-ium iodide hydrochloride. Physical form: colorless amorphous powder; FAB-MS: m/z 750 (M-HCl-I)⁺; Ratio of Retention Time in HPLC: 0.60(see Example D).

### 7.11.

1-[[N-methyl-N-2-(methylamino)acetoxymethyl-5-nitro-phenyl]carbamoyloxy]ethyl-1-[(2R,3R)-2-(2,5-difluorophenyl)-2-hydroxy-3-[4-(4-cyanophenyl)thiazol-2-yl]butyl]-1H-[1,2,4]triazol-4-ium chloride hydrochloride. Physical form: colorless amorphous powder; FAB-MS: m/z 761 (M-HCl-Cl)⁺; Ratio of Retention Time in HPLC: 0.83(see Example D). ¹H-NMR (DMSO): δ 1.10∼1.31 (4H, m), 1.45∼1.65 (2H, m), 1.79∼1.96 (2H, m), 3.08∼3.30 (4H, m), 3.95∼4.25 (4H, m), 4.65∼4.90 (1H, m), 4.99∼5.25 (1H, m), 6.63∼6.89 (1H, m), 7.05∼7.46 (4H, m), 7.78∼8.04 (4H, m), 8.12∼8.39 (4H, m), 8.50 (1H, s). 9.02∼9.38 (1H, m), 9.60 (1H, brs), 10.31∼10.69 (1H. m).
¹H-NMR (DMSO-d6): δ 1.19(3H,d,J=7.3Hz), 1.83-2.76(7H,m), 3.90-4.42(6H,m), 4.81(1H,br.d,J=14.5Hz), 5.10(1H,br.d,J=14.5Hz), 6.25(2H,m), 7.05-7.38(3H,m), 7.93(2H,br.d,J=8.3Hz), 8.21(2H,br.d,J=8.3Hz), 8.46(1H,br.s), 9.11(1H,br.s), 9.20(2H,br.s), 10.2(1H,br.s).

### 7.13.

1-[[N-methyl-N-2-(methylamino)acetoxymethyl-3-fluoro-phenyl]carbamoyloxy]ethyl-1-[(2R,3R)-2-(2,5-difluorophenyl)-2-hydroxy-3-[4-(4-cyanophenyl)thiazol-2-yl]butyl]-1H-[1,2,4]triazol-4-ium chloride hydrochloride. Physical form: colorless amorphous powder; FAB-MS: m/z 734 (M-HCl-Cl) ⁺; Ratio of Retention Time in HPLC: 0.77(see Example D).

### 7.14.

1-[[N-methyl-N-2-(methylamino)acetoxymethyl-5-cyano-phenyl]carbamoyloxy]ethyl-1-[(2R,3R)-2-(2,5-difluorophenyl)-2-hydroxy-3-[4-(4-cyanophenyl)thiazol-2-yl]butyl]-1H-[1,2,4] triazol-4-ium chloride hydrochloride. Physical form: pale yellow powder. LC-MS: m/z 741 (M-HCl-Cl) ⁺; Ratio of Retention Time in HPLC: 0.50(see Example D).

### 7.15.

1-[[N-methyl-N-2-(methylamino)acetoxymethyl-3-chloro-phenyl]carbamoyloxy]ethyl-1-[(2R,3R)-2-(2,5-difluorophenyl)-2-hydroxy-3-[4-(4-cyanophenyl)thiazol-2-yl]butyl]-1H-[1,2,4]triazol-4-ium chloride hydrochloride. Physical form: colorless amorphous powder; FAB-MS: m/z 750 (M-HCl-Cl) ⁺; Ratio of Retention Time in HPLC: 0.88(see Example D).

### 7.16.

1-[[N-methyl-N-2-(methylamino)acetoxymethyl-4-cyano-phenyl]carbamoyloxy]ethyl-1-[(2R,3R)-2-(2,5-difluorophenyl)-2-hydroxy-3-[4-(4-cyanophenyl)thiazol-2-yl]butyl]-1H-[1,2,4]triazol-4-ium chloride hydrochloride. Physical form: colorless amorphous powder; FAB-MS: m/z 741 (M-HCl-Cl)⁺; Ratio of Retention Time in HPLC: 0.82(see Example D). ¹H-NMR (CD₃OD): δ 1.22-2.00(6H,m), 2.73-2.77(6H,m), 3.23-3.34(1H,m), 3.88-4.10(3H,m), 4.28-4.40(1H,m), 5.06-5.24(2H,m), 5.29-5.33(1H,m), 7.00-7.30(3H,m), 7.40-7.56(1H,m), 7.73-7.85(3H,m), 7.86-7.95(1H,m), 8.10-8.22(4H,m), 8.80-9.05(1H,m).

### 7.17.

1-[[N-methyl-N-2-(methylamino)acetoxymethyl-5-trifluoromethyl-phenyl]carbamoyloxy]ethyl-1-[(2R,3R)-2-(2,5-difluorophenyl)-2-hydroxy-3-[4-(4-cyanophenyl)thiazol-2-yl]butyl]-1H-[1,2,4]triazol-4-ium iodide hydrochloride. Physical form: pale yellow powder. LC-MS: m/z 784 (M-HCl-I) ⁺; Ratio of Retention Time in HPLC: 1.09 (see Example D).

### 7.18.

1-[[N-methyl-N-2-(amino)acetoxymethyl-3-chloro-phenyl]carbamoyloxy]ethyl-1-[(2R,3R)-2-(2,5-difluorophenyl)-2-hydroxy-3-[4-(4-cyanophenyl)thiazol-2-yl]butyl]-1H-[1,2,4]triazol-4-ium chloride hydrochloride. Physical form: colorless amorphous powder; FAB-MS: m/z 736 (M-HCl-Cl) ⁺; Ratio of Retention Time in HPLC: 0.83(see Example D).

### 7.19.

1-[[N-ethyl-N-2-(methylamino)acetoxymethyl-3-chloro-phenyl]carbamoyloxy]ethyl-1-[(2R,3R)-2-(2,5-difluorophenyl)-2-hydroxy-3-[4-(4-cyanophenyl)thiazol-2-yl]butyl]-1H-[1,2,4]triazol-4-ium chloride hydrochloride. Physical form: colorless amorphous powder; FAB-MS: m/z 764 (M-HCI-Cl) ⁺; Ratio of Retention Time in HPLC: 0.91(see Example D).

### 7.20.

1-[[N-methyl-N-3-[(amino)acetoxymethyl]pyridin-2-yl]carbamoyloxy]ethyl-1-[(2R,3R)-2-(2,5-difluorophenyl)-2-hydroxy-3-[4-(4-cyanophenyl)thiazol-2-yl]butyl]-1H-[1,2,4]triazol-4-ium chloride hydrochloride. Physical form: colorless amorphous powder; FAB-MS: m/z 703 (M-HCl-Cl)⁺; Ratio of Retention Time in HPLC: 0.70(see Example D).

### 7.21.

1- [[N-methyl-N-2-(methylamino)acetoxymethyl-3-methyl-phenyl]carbamoyloxy] ethyl-1-[(2R,3R)-2-(2,5-difluorophenyl)-2-hydroxy-3-[4-(4-cyanophenyl)thiazol-2-yl]butyl]-1H-[1,2,4]triazol-4-ium iodide hydrochloride. Physical form: colorless amorphous powder; FAB-MS: m/z 730 (M-HCl-I)⁺. ¹H-NMR (DMSO-d6): δ 1.20(3H,d,J=7.3Hz), 1.50-1.86(3H,m), 2.32-2.55(6H,m), 3.12(3H,m), 3.82-4.20(3H,m), 4.75-5.30(4H,m), 6.66-7.38(7H,m), 7.93(2H,br.d,J=8.3Hz), 8.22(2H,br.d,J=8.3Hz), 8.47(1H,br.s), 9.20(1H,m), 10.5(1H,m).

### 7.22.

1-[[N-ethoxycarbonyl-N-2-(methylamino)acetoxymethyl-phenyl]carbamoyloxy]ethyl-1-[(2R,3R)-2-(2,5-difluorophenyl)-2-hydroxy-3-[4-(4-cyanophenyl)thiazol-2-yl]butyl]-1H-[1,2,4]triazol-4-ium chloride hydrochloride. Physical form: pale yellow solid. LC-MS: m/z 774 (M-HCl-Cl)⁺.

### 7.23.

1-[[N-pivaloyl-N-2-(methylamino)acetoxymethyl-phenyl]carbamoyloxy]ethyl-1[(2R,3R)-2-(2,5-difluorophenyl)-2-hydroxy-3-[4-(4-cyanophenyl)thiazol-2-yl]butyl]-1H-[1,2,4]triazol-4-ium chloride hydrochloride. Physical form: pale yellow solid. LC-MS: m/z 786 (M-HCl-Cl)⁺.

### Example A:

### Manufacture of dry ampoules for intramuscular administration:

A lyophilizate of 0.5 g of 1- [[N-methyl-N-3- [(methylamino)acetoxymethyl]pyridin-2-yl]carbamoyloxy]ethyl-1-[(2R,3R)-2-(2,5-difluorophenyl)-2-hydroxy-3-[4-(4-cyanophenyl)thiazol-2-yl]butyl]-1H-[1,2,4]triazol-4-ium chloride hydrochloride is prepared in the usual manner and filled into an ampoule. Prior to the administration the lyophilizate is treated with 2.5 ml of a 2% aqueous lidocaine hydrochloride solution.

### Example B:

Hard gelatin capsules each containing the following ingredients were manufactured in the conventional manner *per se*:

| | |
|---|---|
| a)(1-[[N-methyl-N-3-[(methylamino)acetoxymethyl]pyridin-2-yl]carbamoyloxy]ethyl-1 [(2R,3R)-2-(2,5-difluorophenyl)-2-hydroxy-3-[4-(4-cyanophenyl)thiazol-2-yl]butyl]-1H-[1,2,4]triazol-4-ium chloride hydrochloride | 100mg |
| b) Lactose | 56 mg |
| c) Crystalline Cellulose | 30 mg |
| d) Silicic acid, Light Anhydrous | 10 mg |
| e) Talc | 3 mg |
| f) Magnesium stearate | 1 mg |
| | Total 200 mg |

### Example C:

Tablets each containing the following ingredients were manufactured in the conventional manner *per se*:

| | |
|---|---|
| a) 1-[[N-methyl-N-3-[(methylamino)acetoxymethyl]pyridin-2-yl]carbamoyloxy]ethyl-1-[(2R,3R)-2-(2,5-difluorophenyl)-2-hydroxy-3-[4-(4-cyanophenyl)thiazol-2-yl]butyl]-1H-[1,2,4]triazol-4-ium chloride hydrochloride | 100mg |
| b) Lactose | 60 mg |
| c) Corn starch | 20 mg |
| d) Sodium Starch Glycolate | 10 mg |
| e) Polyvinylpyrrolidone | 6 mg |
| f) Talc | 3 mg |
| g) Magnesium stearate | 1 mg |
| | Total 200 mg |

### Example D:

### HPLC Condition and Ratio of Retention Time of the compound of the general formula (I)

### HPLC Condition

1. Analytical Column: YMC-Pack ODS-AM (AM-313) 5mm, 120A
   250 x 6.0 mmI.D. (No.062505696(W))
   with precolumn:YMC Guardpack ODS-AM,
   5mm, 120A
   10 x 5.0 mml.D. (No.4099(W))
2. Eluent: MeOH/CH₃CN/H₂O/AcOH = 65:10:25:0.1(v/v) containing
   lg/L Sodium 1-Nonanesulfonate: mobile phase A
   or lg/L Sodium 1-Heptanesulfonate: mobile phase B
   or Ig/L Sodium 1-Pentanesulfonate: mobile phase C
   orlg/L Sodium 1-Hexanesulfonate: mobile phase D
3. Flow Rate: 1.1 ml/min
4. Detection: Fluorescence Wavelength: Excitation: 280 nm
   Emission: 350 nm
5. Injection volume: 7 µl

### INSTRUMENTS

1. Pump A: LC-10AS (Shimadzu) Pump B: LC-6A (Shimadzu)
2. Detector: FP-920 (JASCO)
3. Injector: SCL-10A/SIL-10A (Shimadzu) Run time: 18-30 min
4. Switching valve: PT-8000 (TOSOH)
5. Integrator: HPLC Chemstation

### Ratio of Retention Time of the compound of the general formula (I)

### Ratio of Retention Time:

Retention time of compound of general formula(I)/
Retention time of standard compound of Example 5a)
Standard compound of Example 5a):
(2R,3R)-3-[4-(4-cyanophenyl)thiazol-2-yl]-2-(2,5-difluorophenyl)-1-(1H-1,2,4-triazol-1-yl)-butan-2ol.

### Retention Time of Standard compound of Example 5a):

13.2min for mobile phase A
12.6min for mobile phase B
14.1min for mobile phase C
13.0min for mobile phase D

**Table:**

| HPLC results | | |
|---|---|---|
| Example No. | mobile phase | Ratio of retention time |
| 5 | A | 1.79 |
| 7 | B | 0.77 |
| 5.1. | B | 1.10 |
| 5.2. | B | 0.82 |
| 5.7. | B | 1.79 |
| 7.1. | B | 0.78 |
| 7.2. | B | 0.75 |
| 7.3. | B | 0.94 |
| 7.4. | B | 0.83 |
| 7.5. | B | 0.79 |
| 7.6. | B | 0.80 |
| 7.7. | D | 0.70 |
| 7.8. | D | 0.70 |
| 7.9. | B | 0.76 |
| 7.10. | B | 0.60 |
| 7.11. | B | 0.83 |
| 7.13. | B | 0.77 |
| 7.14. | C | 0.50 |
| 7.15. | B | 0.88 |
| 7.16. | B | 0.82 |
| 7.17. | A | 1.09 |
| 7.18. | B | 0.83 |
| 7.19. | B | 0.91 |
| 7.20. | B | 0.70 |

## Claims

1. A compound of the formula (III), 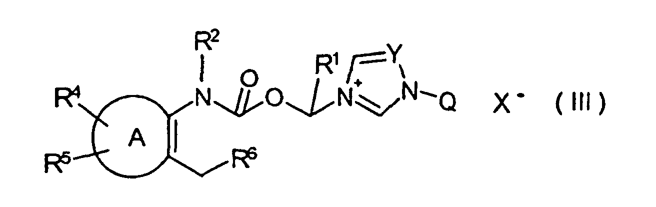 wherein
R¹ is hydrogen or alkyl;
R² is hydrogen, alkyl, alkylcarbonyloxyalkyl, alkoxycarbonyl, alkylcarbonyl, mono- or dialkylaminoalkylcarbonyloxyalkyl;
R⁴ and R⁵ are independently selected from the group consisting of hydrogen, halogen, alkyl, alkoxy, alkylthio, alkylsulfinyl, alkylsulfonyl, carboxy, alkyloxycarbonyl, cyano, trifluoromethyl, trifluormethoxy, nitro, aminosulfonyl or sulfo;
R⁶ is hydroxy, alkoxycarbonylalkylamino, alkoxycarbonylamino, amino, alkylamino, alkylcarbonyloxy, alkoxycarbonylalkylaminoalkylcarbonyloxy, alkoxycarbonylamino-alkylcarbonyloxy, alkylaminoalkylcarbonyloxy, aminoalkylcarbonyloxy, alkylcarbonylamino, alkylcarbonylalkylamino, acyloxy, acylamino, acylalkylamino;
the group 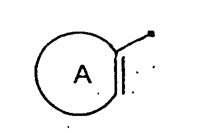 is phenyl or pyridin-2-yl; 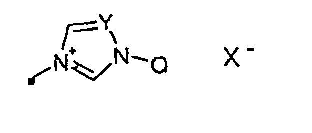 is 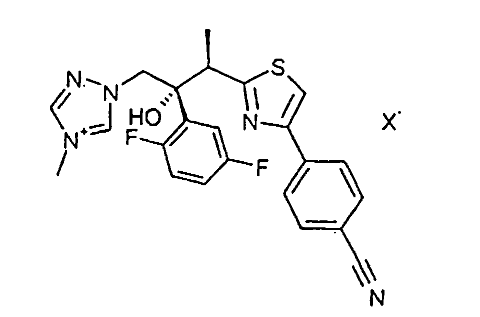 and
X⁻ is a pharmaceutically acceptable anion,
wherein
"acyl" refers to an acyl residue of an amino acid or a group represented by the formula R⁷CO- or (R⁸O)₂PO-, wherein R⁷ is hydrogen, alkoxy, alkyl which may be optionally substituted with carboxy, amino, alkylamino, dialkylamino, or phenyl; and R⁸ is hydrogen or alkyl,
"alkyl" refers to a branched or unbranched saturated hydrocarbon radical having 1 to 6 carbon atom(s),
"alkoxy" refers a straight or branched alkyl-O- chain having 1 to 5 carbon atom(s), "halogen" denotes fluorine, chlorine or bromine, and
"alkylthio" refers to a straight or branched alkyl-S- chain having 1 to 4 carbon atom(s),
as well as pharmaceutically acceptable salts, hydrates or solvates of the compounds of formula (III).

2. Compounds according to claim 1 wherein R¹ is hydrogen or alkyl.

3. Compounds according to claim 1 or 2 wherein R¹ is methyl.

4. Compounds according to any of claims 1 to 3 wherein R² is hydrogen or alkyl.

5. Compounds according to any of claims 1 to 4 wherein R² is alkyl.

6. Compounds according to any of claims 1 to 5 wherein X is a halogen.

7. Compounds according to any of claims 1 to 6 wherein X is chloro.

8. Compounds according to any of claims 1 to 7 wherein R⁴ and R⁵ are independently selected from the group consisting of hydrogen, halogen, alkoxy, cyano, trifluoromethyl, trifluormethoxy and nitro.

9. Compounds according to any of claims 1 to 8 wherein R⁴ and R⁵ are independently selected from the group consisting of hydrogen, halogen and alkoxy.

10. Compounds according to any of claims 1 to 9 wherein R⁴ and R⁵ are hydrogen.

11. Compounds according to any of claims 1 to 10 wherein R⁶ is alkylamino, alkylcarbonyloxy, alkylaminoalkylcarbonyloxy or aminoalkylcarbonyloxy.

12. Compounds according to any of claims 1 to 11 wherein R⁶ is alkylaminoalkylcarbonyloxy.

13. Compounds according to any of claims 1 to 12 wherein the group 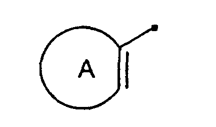 is phenyl or pyridin-2-yl.

14. Compounds according to any of claims 1 to 13 wherein the group 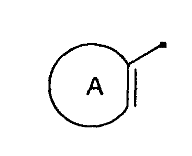 is pyridin-2-yl.

15. Compounds according to any of claims 1 to 13, wherein
R' is alkyl;
R² is alkyl; 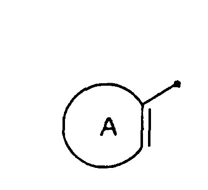 is pyridin-2-yl;
X⁻ is halogen;
R⁴ and R⁵ are hydrogen;
R⁶ is alkylaminoalkylcarbonyloxy;
as well as pharmaceutically acceptable salts, hydrates or solvates thereof.

16. Compounds according to any of claims 1 - 15 selected from the group consisting of
a) [[N-methyl-N-2-(acetoxymethyl)phenyl]carbamoyloxy]methyl-1-[(2R,3R)-2-(2,5-difluorophenyl)-2-hydroxy-3-[4-(4-cyanophenyl)thiazol-2-yl]butyl]-1H-[1,2,4]triazol-4-ium chloride,
b) 1-[[N-methyl-N-2-(isopropylaminomethyl)phenyl]carbamoyloxy]ethyl-1-[(2R,3R)-2-(2,5-difluorophenyl)-2-hydroxy-3-[4-(4-cyanophenyl)thiazol-2-yl]butyl]-1H-[1,2,4]triazol-4-ium chloride hydrochloride,
c) 1-[[N-methyl-N-3-[(methylamino)acetoxymethyl]pyridin-2-yl]carbamoyloxy]ethyl-1-[(2R,3R)-2-(2,5-difluorophenyl)-2-hydroxy-3-[4-(4-cyanophenyl)thiazol-2-yl]butyl]-1H-[1,2,4]triazol-4-ium chloride dihydrochloride,
d) 1-[[N-methyl-N-3-[(methylamino)acetoxymethyl]pyridin-2-yl]carbamoyloxy]ethyl-1-[(2R,3R)-2-(2,5-difluorophenyl)-2-hydroxy-3-[4-(4-cyanophenyl)thiazol-2-yl]butyl]-I H-[1,2,4]triazol-4-ium chloride hydrochloride,
f) [[N-methyl-N-phenyl]carbamoyloxy]methyl-1-[(2R,3R)-2-(2,5-difluorophenyl)-2-hydroxy-3-[4-(4-cyanophenyl)thiazol-2-yl]butyl]-1H-[1,2,4]triazol-4-ium chloride,
g) 1-[[N-methyl-N-3-(acetoxymethyl) pyridin-2-yl]carbamoyloxy]ethyl-1-[(2R,3R)-2-(2,5-difluorophenyl)-2-hydroxy-3-[4-(4-cyanophenyl)thiazol-2-yl]butyl]-1H-[1,2,4]triazol-4-ium chloride hydrochloride,
l) [[N-2-(methyl)phenyl-N-2-(acetoxy)ethyl]carbamoyloxy]methyl-1-[(2R,3R)-2-(2,5-difluorophenyl)-2-hydroxy-3-[4-(4-cyanophenyl)thiazol-2-yl]butyl]-1H-[1,2,4]triazol-4-ium iodide,
m) 1-[[N-2-[(isopropylamino)methyl]phenyl]carbamoyloxy]ethyl-1-[(2R,3R)-2-(2,5-difluorophenyl)-2-hydroxy-3-[4-(4-cyanophenyl)thiazol-2-yl]butyl]-1H-[1,2,4]triazol-4-ium chloride hydrochloride,
n) 1-[[N-2-[(pentan-3-ylamino)methyl]phenyl]carbamoyloxy]ethyl-1-[(2R,3R)-2-(2,5-difluorophenyl)-2-hydroxy-3-[4-(4-cyanophenyl)thiazol-2-yl]butyl]-1H-[1,2,4]triazol-4-ium chloride hydrochloride,
o) 1-[[N-methyl-N-2-[(methylamino)methyl]phenyl]carbamoyloxy]ethyl-1-[(2R,3R)-2-(2,5-difluorophenyl)-2-hydroxy-3-[4-(4-cyanophenyl)thiazol-2-yl]butyl]-1H-[1,2,4]triazol-4-ium chloride hydrochloride,
p) [[N-methyl-N-2-[(methylamino)acetoxymethyl]phenyl]carbamoyloxy]methyl-1-[(2R,3R)-2-(2,5-difluorophenyl)-2-hydroxy-3-[4-(4-cyanophenyl)thiazol-2-yl]butyl]-1H-[1,2,4]triazol-4-ium chloride hydrochloride,
q) 1-[[N-methyl-N-2-[(methylamino)acetoxymethyl]phenyl]carbamoyloxy]ethyl-1-[(2R,3R)-2-(2,5-difluorophenyl)-2-hydroxy-3-[4-(4-cyanophenyl)thiazol-2-yl]butyl]-1H-[1,2,4]triazol-4-ium chloride hydrochloride,
r) 1-[[N-methyl-N-2-(methylamino)acetoxymethyl-4,5-difluorophenyl]carbamoyloxy]-ethyl-1-[(2R,3R)-2-(2,5-difluorophenyl)-2-hydroxy-3-[4-(4-cyanophenyl)thiazol-2-yl]butyl]-1H-[1,2,4]triazol-4-ium chloride hydrochloride,
s) 1-[[N-methyl-N-2-(methylamino)acetoxymethyl-4-fluorophenyl]carbamoyloxy]ethyl-1-[(2R,3R)-2-(2,5-difluorophenyl)-2-hydroxy-3-[4-(4-cyanophenyl)thiazol-2-yl]butyl]-1H-[1,2,4]triazol-4-ium iodide hydrochloride,
t) [[N-methyl-N-2-(methylamino)acetoxymethyl-4,5-dimethoxy-phenyl]carbamoyloxy]-methyl-1-[(2R,3R)-2-(2,5-difluorophenyl)-2-hydroxy-3-[4-(4-cyanophenyl)thiazol-2-yl]butyl]-1H-[1,2,4]triazol-4-ium iodide hydrochloride,
u) 1-[[N-methyl-N-2-(methylamino)acetoxymethyl-5-fluorophenyl]carbamoyloxy]ethyl-1-[(2R,3R)-2-(2,5-difluorophenyl)-2-hydroxy-3-[4-(4-cyanophenyl)thiazol-2-yl]butyl]-1H-[1,2,4]triazol-4-ium chloride hydrochloride,
v) 1-[[N-methyl-N-2-(methylamino)acetoxymethyl-6-methylphenyl]carbamoyloxy]ethyl-1-[(2R,3R)-2-(2,5-difluorophenyl)-2-hydroxy-3-[4-(4-cyanophenyl)thiazol-2-yl]butyl]-1H-[1,2,4]triazol-4-ium iodide hydrochloride,
w) 1-[[N-methyl-N-2-(methylamino)acetoxymethyl-4-chlorophenyl]carbamoyloxy]ethyl-1-[(2R,3R)-2-(2,5-difluorophenyl)-2-hydroxy-3-[4-(4-cyanophenyl)thiazol-2-yl]butyl]-1H-[1,2,4]triazol-4-ium iodide hydrochloride,
x) 1-[[N-(methylamino)acetoxyethyl-N-2,4-difluorophenyl]carbamoyloxy]ethyl-1-[(2R,3R)-2-(2,5-difluorophenyl)-2-hydroxy-3-[4-(4-cyanophenyl)thiazol-2-yl]butyl]-I H-[1,2,4]triazol-4-ium chloride hydrochloride,
y) 1-[[N-methyl-N-2-(methylamino)acetoxymethyl-5-chlorophenyl]carbamoyloxy]ethyl-1-[(2R,3R)-2-(2,5-difluorophenyl)-2-hydroxy-3-[4-(4-cyanophenyl)thiazol-2-yl]butyl]-1H-[1,2,4]triazol-4-ium iodide hydrochloride,
z) 1-[[N-methyl-N-2-(methylamino)acetoxymethyl-5-nitro-phenyl]carbamoyloxy]ethyl-1-[(2R,3R)-2-(2,5-difluorophenyl)-2-hydroxy-3-[4-(4-cyanophenyl)thiazol-2-yl]butyl]-1H-[1,2,4]triazol-4-ium chloride hydrochloride,
bb) 1-[[N-methyl-N-2-(methylamino)acetoxymethyl-3-fluorophenyl]carbamoyloxy]ethyl-1-[(2R,3R)-2-(2,5-difluorophenyl)-2-hydroxy-3-[4-(4-cyanophenyl)thiazol-2-yl]butyl]-1H-[1,2,4]triazol-4-ium chloride hydrochloride,
cc) 1-[[N-methyl-N-2-(methylamino)acetoxymethyl-5-cyanophenyl]carbamoyloxy]ethyl-1-[(2R,3R)-2-(2,5-difluorophenyl)-2-hydroxy-3-[4-(4-cyanophenyl)thiazol-2-yl]butyl]-1H-[1,2,4]triazol-4-ium chloride hydrochloride,
dd) 1-[[N-methyl-N-2-(methylamino)acetoxymethyl-3-chlorophenyl]carbamoyloxy]ethyl-1-[(2R,3R)-2-(2,5-difluorophenyl)-2-hydroxy-3-[4-(4-cyanophenyl)thiazol-2-yl]butyl]-1H-[1,2,4]triazol-4-ium chloride hydrochloride,
ee) 1-[[N-methyl-N-2-(methylamino)acetoxymethyl-4-cyanophenyl]carbamoyloxy]ethyl-1-[(2R,3R)-2-(2,5-difluorophenyl)-2-hydroxy-3-[4-(4-cyanophenyl) thiazol-2-yl]butyl]-1H-[1,2,4]triazol-4-ium chloride hydrochloride,
ff) 1-[[N-methyl-N-2-(methylamino)acetoxymethyl-5-trifluoromethyl-phenyl]carbamoyloxy]ethyl-1-[(2R,3R)-2-(2,5-difluorophenyl)-2-hydroxy-3-[4-(4-cyanophenyl)thiazol-2-yl)butyl]-1H-[1,2,4]triazol-4-ium iodide hydrochloride,
gg) 1-[[N-methyl-N-2-(amino)acetoxymethyl-3-chloro-phenyl]carbamoyloxy]ethyl-1-[(2R,3R)-2-(2,5-difluorophenyl)-2-hydroxy-3-[4-(4-cyanophenyl)thiazol-2-yl]butyl]-11-3-[1,2,4]triazol-4-ium chloride hydrochloride,
hh) 1-[[N-ethyl-N-2-(methylamino)acetoxymethyl-3-chloro-phenyl]carbamoyloxy]ethyl-1-[(2R,3R)-2-(2,5-difluorophenyl)-2-hydroxy-3-[4-(4-cyanophenyl)thiazol-2-yl]butyl]-1H-[1,2,4]triazol-4-ium chloride hydrochloride,
ii) 1-[[N-methyl-N-3-[(amino)acetoxymethyl]pyridin-2-yl]carbamoyloxy]ethyl-1-[(2R,3R)-2-(2,5-difluorophenyl)-2-hydroxy-3-[4-(4-cyanophenyl)thiazol-2-yl]butyl]-I H-[1,2,4]triazol-4-ium chloride hydrochloride,
jj) 1-[[N-methyl-N-2-(methylamino)acetoxymethyl-3-methylphenyl]carbamoyloxy]ethyl-1-[(2R,3R)-2-(2,5-difluorophenyl)-2-hydroxy-3-[4-(4-cyanophenyl)thiazol-2-yl]butyl]-1H-[1,2,4]triazol-4-ium iodide hydrochloride,
kk) 1-[[N-ethoxycarbonyl-N-2-(methylamino)acetoxymethyl-phenyl]carbamoyloxy]ethyl-1-[(2R,3R)-2-(2,5-difluorophenyl)-2-hydroxy-3-[4-(4-cyanophenyl)thiazol-2-yl]butyl]-1H-[1,2,4]triazol-4-ium chloride hydroehloride,
II) 1-[[N-pivaloyl-N-2-(methylamino)acetoxymethyl-phenyl]carbamoyloxy]ethyl-1-[(2R,3R)-2-(2,5-difluorophenyl)-2-hydroxy-3-[4-(4-cyanophenyl)thiazol-2-yl]butyl]-1H-[1,2,4]triazol-4-ium chloride hydrochloride.

17. A compound according to any of claims 1 - 16 which is 1-[[N-methyl-N-3-[(methylamino)acetoxymethyl]pyridin-2-yl]carbamoyloxy]ethyl-1-[(2R,3R)-2-(2,5-difluorophenyl)-2-hydroxy-3-[4-(4-cyanophenyl)thiazol-2-yl]butyl]-1H-[1,2,4]triazol-4-ium chloride hydrochloride.

18. A compound of formula (V) which is 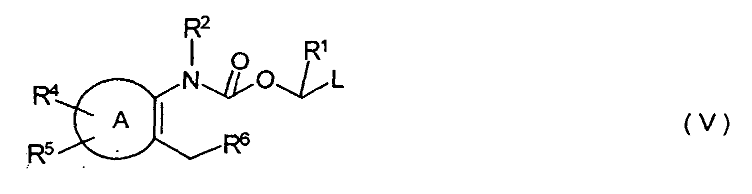 wherein R¹, R², R⁴, R⁵, R⁶ and the group 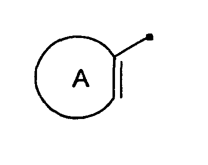 are as defined in any of claims 1 - 17 and L is a leaving group.

19. A process for the manufacture of a compound of the general formula (III) as defined in claim 1, which comprises reacting an azole compound possessing antifungal activity of the general formula (II) 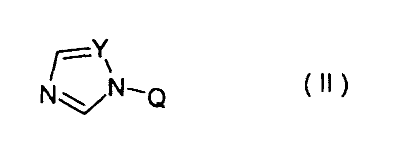 wherein Y and Q are as defined in claim 1, with a compound of general formula (V) as defined in claim 18.

20. Compounds according to any of claims 1 - 17 as obtained by a process according to claim 19.

21. A pharmaceutical composition, in particular for use as an antifungal, comprising a compound as defined in any one of claims I to 17 and a pharmaceutically acceptable carrier.

22. The compounds as defined in any one of claims 1 to 17 for use as therapeutic active substances, in particular as antifungallly active substances.

23. The use of a compound as defined in any one of claims 1 to 17 for the preparation of a medicament for the prophylaxis and/or treatment of fungal infections.

## Patentansprüche

1. Eine Verbindung der Formel (III), 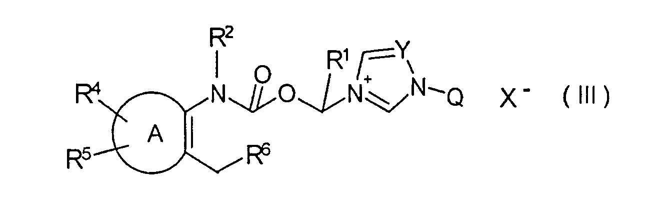 wobei
R¹ Wasserstoff oder Alkyl ist;
R² Wasserstoff, Alkyl, Alkylcarbonyloxyalkyl, Alkoxycarbonyl, Alkylcarbonyl, Mono- oder Dialkylaminoalkylcarbonyloxyalkyl ist;
R⁴ und R⁵ unabhängig ausgewählt werden aus der Gruppe, bestehend aus Wasserstoff, Halogen, Alkyl, Alkoxy, Alkylthio, Alkylsulfinyl, Alkylsulfonyl, Carboxy, Alkyloxycarbonyl, Cyano, Trifluormethyl, Trifluormethoxy, Nitro, Aminosulfonyl oder Sulfo;
R⁶ Hydroxy, Alkoxycarbonylalkylamino, Alkoxycarbonylamino, Amino, Alkylamino, Alkylcarbonyloxy, Alkoxycarbonylalkylaminoalkylcarbonyloxy, Alkoxycarbonylaminoalkylcarbonyloxy, Alkylaminoalkylcarbonyloxy, Aminoalkylcarbonyloxy, Alkylcarbonylamino, Alkylcarbonylalkylamino, Acyloxy, Acylamino, Acylalkylamino ist; die Gruppe 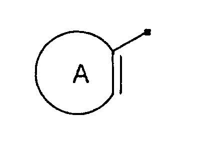 Phenyl oder Pyridin-2-yl ist; 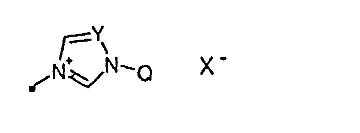 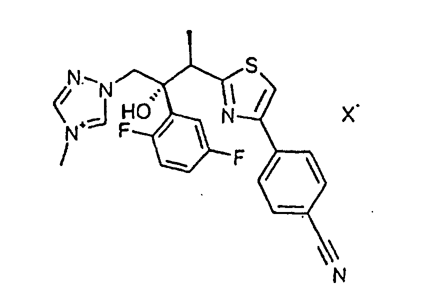 ist und
X⁻ ein pharmazeutisch verträgliches Anion ist,
wobei
"Acyl" sich auf einen Acylrest einer Aminosäure oder eine Gruppe, die durch die Formel R⁷CO- oder (R⁸O)₂PO- dargestellt wird, bezieht, wobei R⁷ Wasserstoff, Alkoxy, Alkyl, welches gegebenenfalls substituiert sein kann mit Carboxy, Amino, Alkylamino, Dialkylamino, oder Phenyl ist; und R⁸ Wasserstoff oder Alkyl ist, "Alkyl" sich auf eine verzweigte oder unverzweigte gesättigte Kohlenwasserstoffgruppe mit 1 bis 6 Kohlenstoffatom(en) bezieht,
"Alkoxy" sich auf eine gerade oder verzweigte Alkyl-O-Kette mit 1 bis 5 Kohlenstoffatom(en) bezieht,
"Halogen" Fluor, Chlor oder Brom bezeichnet und
"Alkylthio" sich auf eine gerade oder verzweigte Alkyl-S-Kette mit 1 bis 4 Kohlenstoffatom(en) bezieht,
wie auch pharmazeutisch verträgliche Salze, Hydrate oder Solvate der Verbindungen der Formel (III).

2. Verbindungen gemäss Anspruch 1, wobei R¹ Wasserstoff oder Alkyl ist.

3. Verbindungen gemäss Anspruch 1 oder 2, wobei R¹ Methyl ist.

4. Verbindungen gemäss irgendeinem der Ansprüche 1 bis 3, wobei R² Wasserstoff oder Alkyl ist.

5. Verbindungen gemäss irgendeinem der Ansprüche 1 bis 4, wobei R² Alkyl ist.

6. Verbindungen gemäss irgendeinem der Ansprüche 1 bis 5, wobei X ein Halogen ist.

7. Verbindungen gemäss irgendeinem der Ansprüche 1 bis 6, wobei X Chlor ist.

8. Verbindungen gemäss irgendeinem der Ansprüche 1 bis 7, wobei R⁴ und R⁵ unabhängig ausgewählt werden aus der Gruppe, bestehend aus Wasserstoff, Halogen, Alkoxy, Cyano, Trifluormethyl, Trifluormethoxy und Nitro.

9. Verbindungen gemäss irgendeinem der Ansprüche 1 bis 8, wobei R⁴ und R⁵ unabhängig ausgewählt werden aus der Gruppe, bestehend aus Wasserstoff, Halogen und Alkoxy.

10. Verbindungen gemäss irgendeinem der Ansprüche 1 bis 9, wobei R⁴ und R⁵ Wasserstoff sind.

11. Verbindungen gemäss irgendeinem der Ansprüche 1 bis 10, wobei R⁶ Alkylamino, Alkylcarbonyloxy, Alkylaminoalkylcarbonyloxy oder Aminoalkylcarbonyloxy ist.

12. Verbindungen gemäss irgendeinem der Ansprüche 1 bis 11, wobei R⁶ Alkylaminoalkylcarbonyloxy ist.

13. Verbindungen gemäss irgendeinem der Ansprüche 1 bis 12, wobei die Gruppe 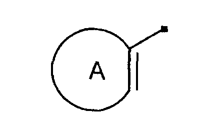 Phenyl oder Pyridin-2-yl ist.

14. Verbindungen gemäss irgendeinem der Ansprüche 1 bis 13, wobei die Gruppe 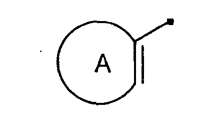 Pyridin-2-yl ist.

15. Verbindungen gemäss irgendeinem der Ansprüche 1 bis 13, wobei
R¹ Alkyl ist;
R² Alkyl ist; 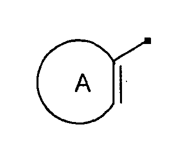 Pyridin-2-yl ist;
X⁻ Halogen ist;
R⁴ und R⁵ Wasserstoff sind;
R⁶ Alkylaminoalkylcarbonyloxy ist;
wie auch pharmazeutisch verträgliche Salze, Hydrate oder Solvate von diesen.

16. Verbindungen gemäss irgendeinem der Ansprüche 1 - 15, die ausgewählt sind aus der Gruppe, bestehend aus
a) [[N-Methyl-N-2-(acetoxymethyl)phenyl]carbamoyloxy]methyl-1-[(2R,3R)-2-(2,5-difluorphenyl)-2-hydroxy-3-[4-(4-cyanophenyl)thiazol-2-yl]butyl]-1H-[1,2,4]triazol-4-iumchlorid,
b) 1-[[N-Methyl-N-2-(isopropylaminomethyl)phenyl]carbamoyloxy]ethyl-1-[(2R,3R)-2-(2,5-difluorphenyl)-2-hydroxy-3-[4-(4-cyanophenyl)thiazol-2-yl]butyl]-1H-[1,2,4]-triazol-4-iumchlorid-hydrochlorid,
c) 1-[[N-Methyl-N-3-[(methylamino)acetoxymethyl]pyridin-2-yl]carbamoyloxy]ethyl-1-[(2R,3R)-2-(2,5-difluorphenyl)-2-hydroxy-3-[4-(4-cyanophenyl)thiazol-2-yl]butyl]-1 H-[ 1,2,4]triazol-4-iumchlorid-dihydrochlorid,
d) 1-[[N-Methyl-N-3-[(methylamino)acetoxymethyl]pyridin-2-yl]carbamoyloxy]ethyl-1-[(2R,3R)-2-(2,5-difluorphenyl)-2-hydroxy-3-[4-(4-cyanophenyl)thiazol-2-yl]butyl]-1 H-[1,2,4]triazol-4-iumchlorid-hydrochlorid,
f) [[N-Methyl-N-phenyl]carbamoyloxy]methyl-1-[(2R,3R)-2-(2,5-difluorphenyl)-2-hydroxy-3-[4-(4-cyanophenyl)thiazol-2-yl]butyl]-1H-[ 1,2,4]triazol-4-iumchlorid,
g) 1-[[N-Methyl-N-3-(acetoxymethyl)pyridin-2-yl]carbamoyloxy]ethyl-1-[(2R,3R)-2-(2,5-difluorphenyl)-2-hydroxy-3-[4-(4-cyanophenyl)thiazol-2-yl]butyl]-1H-[ 1,2,4]-triazol-4-iumchlorid-hydrochlorid,
l) [[N-2-(Methyl)phenyl-N-2-(acetoxy)ethyl]carbamoyloxy]methyl-1-[(2R,3R)-2-(2,5-difluorphenyl)-2-hydroxy-3-[4-(4-cyanophenyl)thiazol-2-yl]butyl]-1H-[1,2,4]triazol-4-iumiodid,
m) 1-[[N-2-[(Isopropylamino)methyl]phenyl]carbamoyloxy]ethyl-1-[(2R,3R)-2-(2,5-difluorphenyl)-2-hydroxy-3-[4-(4-cyanophenyl)thiazol-2-yl]butyl]-1H-[1,2,4]triazol-4-iumchlorid-hydrochlorid,
n) 1-[[N-2-[(Pentan-3-ylamino)methyl]phenyl]carbamoyloxy]ethyl-1-[(2R,3R)-2-(2,5-difluorphenyl)-2-hydroxy-3-[4-(4-cyanophenyl)thiazol-2-yl]butyl]-1H-[1,2,4]triazol-4-iumchlorid-hydrochlorid,
o) 1-[[N-Methyl-N-2-[(methylamino)methyl]phenyl]carbamoyloxy]ethyl-1-[(2R,3R)-2-(2,5-difluorphenyl)-2-hydroxy-3-[4-(4-cyanophenyl)thiazol-2-yl]butyl]-1H-[1,2,4]-triazol-4-iumchlorid-hydrochlorid,
p) [[N-Methyl-N-2-[(methylamino)acetoxymethyl]phenyl]carbamoyloxy]methyl-1-[(2R,3R)-2-(2,5-difluorphenyl)-2-hydroxy-3-[4-(4-cyanophenyl)thiazol-2-yl]butyl]-1 H-[1,2,4]triazol-4-iumchlorid-hydrochlorid,
q) 1-[[N-Methyl-N-2-[(methylamino)acetoxymethyl]phenyl]carbamoyloxy]ethyl-1-[(2R,3R)-2-(2,5-difluorphenyl)-2-hydroxy-3-[4-(4-cyanophenyl)thiazol-2-yl]butyl]-1 H-[ 1,2,4]triazol-4-iumchlorid-hydrochlorid,
r) 1-[[N-Methyl-N-2-(methylamino)acetoxymethyl-4,5-difluorphenyl]carbamoyloxy]-ethyl-1-[(2R,3R)-2-(2,5-difluorphenyl)-2-hydroxy-3-[4-(4-cyanophenyl)thiazol-2-yl]butyl]-1H-[1,2,4]triazol-4-iumchlorid-hydrochlorid,
s) 1-[[N-Methyl-N-2-(methylamino)acetoxymethyl-4-fluorphenyl]carbamoyloxy]ethyl-1-[(2R,3R)-2-(2,5-difluorphenyl)-2-hydroxy-3-[4-(4-cyanophenyl)thiazol-2-yl]butyl]-1 H-[ 1,2,4]triazol-4-iumiodid-hydrochlorid,
t) [[N-Methyl-N-2-(methylamino)acetoxymethyl-4,5-dimethoxy-phenyl]carbamoyloxy]-methyl-1-[(2R,3R)-2-(2,5-difluorphenyl)-2-hydroxy-3-[4-(4-cyanophenyl)thiazol-2-yl]butyl]-1H-[1,2,4]triazol-4-iumiodid-hydrochlorid,
u) 1-[[N-Methyl-N-2-(methylamino)acetoxymethyl-5-fluorphenyl]carbamoyloxy]ethyl-1-[(2R,3R)-2-(2,5-difluorphenyl)-2-hydroxy-3-[4-(4-cyanophenyl)thiazol-2-yl]butyl]-1H-[ 1,2,4]triazol-4-iumchlorid-hydrochlorid,
v) 1-[[N-Methyl-N-2-(methylamino)acetoxymethyl-6-methylphenyl]carbamoyloxy]-ethyl-1-[(2R,3R)-2-(2,5-difluorphenyl)-2-hydroxy-3-[4-(4-cyanophenyl)thiazol-2-yl]butyl]-1H-[1,2,4]triazol-4-iumiodid-hydrochlorid,
w) 1-[[N-Methyl-N-2-(methylamino)acetoxymethyl-4-chlorphenyl]carbamoyloxy]ethyl-1-[(2R,3R)-2-(2,5-difluorphenyl)-2-hydroxy-3-[4-(4-cyanophenyl)thiazol-2-yl]butyl]-1H-[1,2,4]triazol-4-iumiodid-hydrochlorid,
x) 1-[[N-(Methylamino)acetoxyethyl-N-2,4-difluorphenyl]carbamoyloxy]ethyl-1-[(2R,3R)-2-(2,5-difluorphenyl)-2-hydroxy-3-[4-(4-cyanophenyl)thiazol-2-yl]butyl]-1H-[1,2,4]triazol-4-iumchlorid-hydrochlorid,
y) 1-[[N-Methyl-N-2-(methylamino)acetoxymethyl-5-chlorphenyl]carbamoyloxy]ethyl-1-[(2R,3R)-2-(2,5-difluorphenyl)-2-hydroxy-3-[4-(4-cyanophenyl)thiazol-2-yl]butyl]-1H-[1,2,4]triazol-4-iumiodid-hydrochlorid,
z) 1-[[N-Methyl-N-2-(methylamino)acetoxymethyl-5-nitro-phenyl]carbamoyloxy]ethyl-1-[(2R,3R)-2-(2,5-difluorphenyl)-2-hydroxy-3-[4-(4-cyanophenyl)thiazol-2-yl]butyl]-1H-[1,2,4]triazol-4-iumchlorid-hydrochlorid,
bb) 1-[[N-Methyl-N-2-(methylamino)acetoxymethyl-3-fluorphenyl]carbamoyloxy]ethyl-1-[(2R,3R)-2-(2,5-difluorphenyl)-2-hydroxy-3-[4-(4-cyanophenyl)thiazol-2-yl]butyl]-1H-[1,2,4]triazol-4-iumchlorid-hydrochlorid,
cc) 1-[[N-Methyl-N-2-(methylamino)acetoxymethyl-5-cyanophenyl]carbamoyloxy]ethyl-1-[(2R,3R)-2-(2,5-difluorphenyl)-2-hydroxy-3-[4-(4-cyanophenyl)thiazol-2-yl]butyl]-1H-[1,2,4]triazol-4-iumchlorid-hydrochlorid,
dd) 1-[[N-Methyl-N-2-(methylamino)acetoxymethyl-3-chlorphenyl]carbamoyloxy]ethyl-1-[(2R,3R)-2-(2,5-difluorphenyl)-2-hydroxy-3-[4-(4-cyanophenyl)thiazol-2-yl]butyl]-1H-[1,2,4]triazol-4-iumchlorid-hydrochlorid,
ee) 1-[[N-Methyl-N-2-(methylamino)acetoxymethyl-4-cyanophenyl]carbamoyloxy]ethyl-1-[(2R,3R)-2-(2,5-difluorphenyl)-2-hydroxy-3-[4-(4-cyanophenyl)thiazol-2-yl]butyl]-1H-[1,2,4]triazol-4-iumchlorid-hydrochlorid,
ff) 1-[[N-Methyl-N-2-(methylamino)acetoxymethyl-5-trifluormethyl-phenyl]carbamoyloxy]ethyl-1-[(2R,3R)-2-(2,5-difluorphenyl)-2-hydroxy-3-[4-(4-cyanophenyl)thiazol-2-yl]butyl]-1H-[1,2,4]triazol-4-iumiodid-hydrochlorid,
gg) 1-[[N-Methyl-N-2-(amino)acetoxymethyl-3-chlorphenyl]carbamoyloxy]ethyl-1-[(2R,3R)-2-(2,5-difluorphenyl)-2-hydroxy-3-[4-(4-cyanophenyl)thiazol-2-yl]butyl]-1H-[1,2,4]triazol-4-iumchlorid-hydrochlorid,
hh) 1-[[N-Ethyl-N-2-(methylamino)acetoxymethyl-3-chlorphenyl]carbamoyloxy]ethyl-1-[(2R,3R)-2-(2,5-difluorphenyl)-2-hydroxy-3-[4-(4-cyanophenyl)thiazol-2-yl]butyl]-1H-[1,2,4]triazol-4-iumchlorid-hydrochlorid,
ii) 1-[[N-Methyl-N-3-[(amino)acetoxymethyl]pyridin-2-yl]carbamoyloxy]ethyl-1-[(2R,3R)-2-(2,5-difluorphenyl)-2-hydroxy-3-[4-(4-cyanophenyl)thiazol-2-yl]butyl]-1H-[1,2,4]triazol-4-iumchlorid-hydrochlorid,
jj) 1-[[N-Methyl-N-2-(methylamino)acetoxymethyl-3-methylphenyl]carbamoyloxy]-ethyl-1-[(2R,3R)-2-(2,5-difluorphenyl)-2-hydroxy-3-[4-(4-cyanophenyl)thiazol-2-yl]butyl]-1H-[1,2,4]triazol-4-iumiodid-hydrochlorid,
kk) 1-[[N-Ethoxycarbonyl-N-2-(methylamino)acetoxymethylphenyl]carbamoyloxy]ethyl-1-[(2R,3R)-2-(2,5-difluorphenyl)-2-hydroxy-3-[4-(4-cyanophenyl)thiazol-2-yl]butyl]-1H-[1,2,4]triazol-4-iumchlorid-hydrochlorid,
ll) 1-[[N-Pivaloyl-N-2-(methylamino)acetoxymethylphenyl]carbamoyloxy]ethyl-1-[(2R,3R)-2-(2,5-difluorphenyl)-2-hydroxy-3-[4-(4-cyanophenyl)thiazol-2-yl]butyl]-1H-[1,2,4]triazol-4-iumchlorid-hydrochlorid.

17. Eine Verbindung gemäss irgendeinem der Ansprüche 1 - 16, welche 1-[[N-Methyl-N-3-[(methylamino)acetoxymethyl]pyridin-2-yl]carbamoyloxy]ethyl-1-[(2R,3R)-2-(2,5-difluorphenyl)-2-hydroxy-3-[4-(4-cyanophenyl)thiazol-2-yl]butyl]-1H-[1,2,4]triazol-4-iumchlorid-hydrochlorid ist.

18. Eine Verbindung der Formel (V), welche 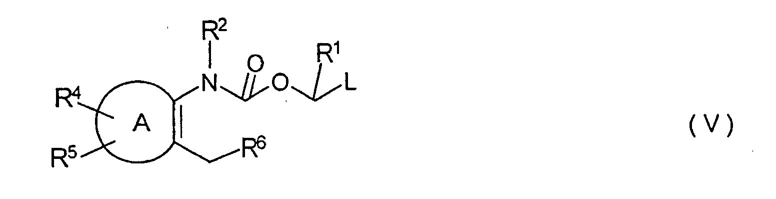 ist, wobei R¹, R², R⁴, R⁵, R⁶ und die Gruppe 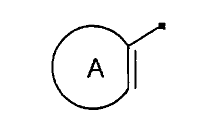 wie in irgendeinem der Ansprüche 1 - 17 definiert sind und L eine Abgangsgruppe ist.

19. Ein Verfahren zur Herstellung einer Verbindung der allgemeinen Formel (III) wie in Anspruch 1 definiert, welches das Umsetzen einer Azolverbindung, die antifungale Aktivität besitzt, mit der allgemeinen Formel (II) 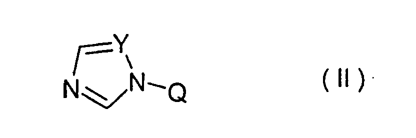 wobei Y und Q wie in Anspruch 1 definiert sind, mit einer Verbindung der allgemeinen Formel (V) wie in Anspruch 18 definiert umfasst.

20. Verbindungen gemäss irgendeinem der Ansprüche 1 - 17, wie diese durch ein Verfahren gemäss Anspruch 19 erhalten werden.

21. Eine pharmazeutische Zusammensetzung, insbesondere zur Verwendung als ein antifungales Mittel, welche eine Verbindung wie in irgendeinem der Ansprüche 1 bis 17 definiert und einen pharmazeutisch verträglichen Träger umfasst.

22. Die Verbindungen, wie sie in irgendeinem der Ansprüche 1 bis 17 definiert sind, zur Verwendung als therapeutisch aktive Substanzen, insbesondere als antifungal aktive Substanzen.

23. Die Verwendung einer Verbindung, wie sie in irgendeinem der Ansprüche 1 bis 17 definiert ist, zur Herstellung eines Medikaments zur Prophylaxe und/oder Behandlung von Pilzinfektionen.

## Revendications

1. Composé de formule (III), 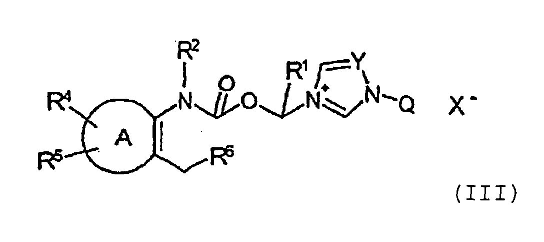 dans laquelle
R¹ est un hydrogène ou un alkyle ;
R² est un hydrogène, un alkyle, un alkylcarbonyloxyalkyle, un alcoxycarbonyle, un alkylcarbonyle, un mono- ou dialkylaminoalkylcarbonyloxyalkyle ;
R⁴ et R⁵ sont indépendamment sélectionnés parmi le groupe constitué d'hydrogène, halogène, alkyle, alcoxy, alkylthio, alkylsulfinyle, alkylsulfonyle, carboxy, alkyloxycarbonyle, cyano, trifluorométhyle, trifluorométhoxy, nitro, aminosulfonyle ou sulfo ;
R⁶ est un hydroxy, alcoxycarbonylalkylamino, alcoxycarbonylamino, amino, alkylamino, alkylcarbonyloxy, alcoxycarbonylalkylaminoalkylcarbonyloxy, alcoxycarbonylaminoalkylcarbonyloxy, alkylaminoalkylcarbonyloxy, aminoalkylcarbonyloxy, alkylcarbonylamino, alkylcarbonylalkylamino, acyloxy, acylamino, acylalkylamino ;
le groupe 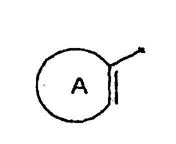 est un phényle ou un pyridin-2-yle ; 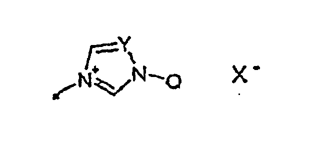 est 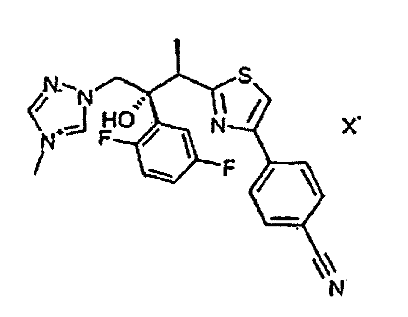 et
X⁻ est un anion pharmaceutiquement acceptable,
dans laquelle
"acyle" fait référence à un résidu acyle d'un acide aminé ou d'un groupe représenté par la formule R⁷CO- ou (R⁸O)₂PO-, dans laquelle R⁷ est un hydrogène, alcoxy, alkyle qui peut être facultativement substitué avec un carboxy, amino, alkylamino, dialkylamino ou phényle ; et R⁸ est un hydrogène ou un alkyle,
"alkyle" fait référence à un radical d'hydrocarbure saturé ramifié ou non ramifié ayant de 1 à 6 atomes de carbone,
"alcoxy" fait référence à une chaîne -O-alkyle linéaire ou ramifiée ayant 1 à 5 atomes de carbone,
"halogène" représente un fluor, un chlore ou un brome et
"alkylthio" fait référence à une chaîne-S-alkyle linéaire ou ramifiée ayant 1 à 4 atomes de carbone,
tout comme les sels, hydrates ou solvates pharmaceutiquement acceptables des composés de formule (III).

2. Composés selon la revendication 1, dans lesquels R¹ est un hydrogène ou un alkyle.

3. Composés selon la revendication 1 ou 2, dans lesquels R¹ est un méthyle.

4. Composés selon l'une quelconque des revendications 1 à 3, dans lesquels R² est un hydrogène ou un alkyle.

5. Composés selon l'une quelconque des revendications 1 à 4, dans lesquels R² est un alkyle.

6. Composés selon l'une quelconque des revendications 1 à 5, dans lesquels X est un halogène.

7. Composés selon l'une quelconque des revendications 1 à 6, dans lesquels X est un chloro.

8. Composés selon l'une quelconque des revendications 1 à 7, dans lesquels R⁴ et R⁵ sont indépendamment sélectionnés parmi le groupe constitué d'un hydrogène, halogène, alcoxy, cyano trifluorométhyle trifluorométhoxy et nitro.

9. Composés selon l'une quelconque des revendications 1 à 8, dans lesquels R⁴ et R⁵ sont indépendamment sélectionnés parmi le groupe constitué d'un hydrogène, halogène et alcoxy.

10. Composés selon l'une quelconque des revendications 1 à 9, dans lesquels R⁴ et R⁵ sont un hydrogène.

11. Composés selon l'une quelconque des revendications 1 à 10, dans lesquels R⁶ est un alkylamino, alkylcarbonyloxy, alkylaminoalkylcarbonyloxy ou aminoalkylcarbonyloxy.

12. Composés selon l'une quelconque des revendications 1 à 11, dans lesquels R⁶ est un alkylaminoalkylcarbonyloxy.

13. Composés selon l'une quelconque des revendications 1 à 12 dans lesquels le groupe 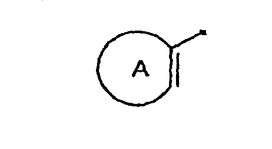 est un phényle ou un pyridin-2-yle.

14. Composés selon l'une quelconque des revendications 1 à 13, dans lesquels le groupe 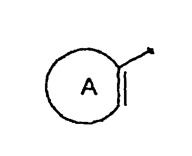 est un pyrdin-2-yle.

15. Composés selon l'une quelconque des revendications 1 à 13, dans lesquels R¹ est un alkyle ;
R² est un alkyle ; 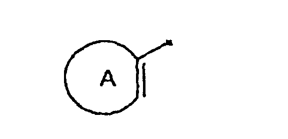 est pyridin-2-yle ;
X⁻ est un halogène ;
R⁴ et R⁵ sont un hydrogène ;
R⁶ est un alkylaminoalkylcarbonyloxy ;
tout comme leurs sels, hydrates ou solvates pharmaceutiquement acceptables.

16. Composés selon l'une quelconque des revendications 1 à 15, sélectionnés parmi le groupe constitué de
a) chlorure de [[N-méthyl-N-2-(acétoxyméthyl)phényl]carbamoyloxy]méthyl-1-[(2R,3R)-2-(2,5-difluorophényl)-2-hydroxy-3-[4-(4-cyanophényl)thiazol-2-yl]butyl]-1H-[1,2,4]triazol-4-ium,
b) chlorhydrate du chlorure de 1-[[N-méthyl-N-2-(isopropylaminométhyl)phényl]carbamoyloxy]éthyl-1-[(2R,3R)-2-(2,5-difluorophényl)-2-hydroxy-3-[4-(4-cyanophényl)thiazol-2-yl]butyl]-1H-[1,2,4]triazol-4-ium,
c) dichlorhydrate du chlorure de 1-[[N-méthyl-N-3-[(méthylamino)acétoxyméthyl]pyridin-2-yl]carbamoyloxy]éthyl-1-[(2R,3R)-2-(2,5-difluorophényl)-2-hydroxy-3-[4-(4-cyanophényl)thiazol-2-yl]butyl]-1H-[1,2,4]triazol-4-ium,
d) chlorhydrate du chlorure de 1-[[N-méthyl-N-3-[(méthylamino)acétoxyméthyl]pyridin-2-yl]carbamoyloxy]éthyl-1-[(2R,3R)-2-(2,5-difluorophényl)-2-hydroxy-3-[4-(4-cyanophényl)thiazol-2-yl]butyl]-1H-[1,2,4]triazol-4-ium,
f) chlorure de [[N-méthyl-N-phényl]carbamoyloxy]méthyl-1-[(2R,3R)-2-(2,5-difluorophényl)-2-hydroxy-3-[4-(4-cyanophényl)thiazol-2-yl]butyl]-1H-[1,2,4]triazol-4-ium,
g) chlorhydrate du chlorure de 1-[[N-méthyl-N-3-(acétoxyméthyl) pyridin-2-yl]carbamoyloxy]éthyl-1-[(2R,3R)-2-(2,5-difluorophényl)-2-hydroxy-3-[4-(4-cyanophényl)thiazol-2-yl]butyl]-1H-[1,2,4]triazol-4-ium,
l) iodure de [[N-2-(méthyl)phényl-N-2-(acétoxy)éthyl]carbamoyloxy]méthyl-1-[(2R,3R)-2-(2,5-difluorophényl)-2-hydroxy-3-[4-(4-cyanophényl)thiazol-2-yl]butyl)-1H-[1,2,4]triazol-4-ium,
m) chlorhydrate du chlorure de 1-[[N-2-[(isopropylamino)méthyl]phényl]carbamoyloxy]-éthyl-1-[(2R,3R)-2-(2,5-difluorophényl)-2-hydroxy-3-[4-(4-cyanophényl)thiazol-2-yl]butyl]-1H-[1,2,4]triazoI-4-ium,
n) chlorhydrate du chlorure de 1-[[N-2-[(pentan-3-ylamino)méthyl]phényl]carbamoyloxy]éthyl-1-[(2R,3R)-2-(2,5-difluorophényl)-2-hydroxy-3-[4-(4-cyanophényl)thiazol-2-yl]butyl]-1H-[1,2,4]triazol-4-ium,
o) chlorhydrate du chlorure de 1-[[N-méthyl-N-2-[(méthylamino)méthyl]phényl]carbamoyloxy]éthyl-1-[(2R,3R)-2-(2,5-difluorophényl)-2-hydroxy-3-[4-(4-cyanophényl)thiazol-2-yl]butyl]-1H-[1,2,4]triazol-4-ium,
p) chlorhydrate du chlorure de [[N-méthyl-N-2-[(méthylamino)acétoxyméthyl]phényl]carbamoyloxy] méthyl-1-[(2R,3R)-2-(2,5-difluorophényl)-2-hydroxy-3-[4-(4-cyanophényl)thiazol-2-yl]butyl]-1H-[1,2,4]triazol-4-ium,
q) chlorhydrate du chlorure de 1-[[N-méthyl-N-2-[(méthylamino)acétoxyméthyl]phényl]carbamoyloxy] éthyl-1-[(2R,3R)-2-(2,5-difluorophényl)-2-hydroxy-3-[4-(4-cyanophényl)thiazol-2-yl]butyl]-1H-[1,2,4]triazol-4-ium,
r) chlorhydrate du chlorure de 1-[[N-méthyl-N-2-(méthylamino) acétoxyméthyl-4,5-difluorophényl]carbamoyloxy]-éthyl-1-[(2R,3R)-2-(2, 5-difluorophényl) -2-hydroxy-3- [4-(4-cyanophényl)thiazol-2-yl]butyl]-1H-[1,2,4]triazol-4-ium,
s) chlorhydrate de l'iodure de 1-[[N-méthyl-N-2-(méthylamino) acétoxyméthyl-4-fluorophényl]carbamoyloxy]éthyl-1-[(2R,3R)-2-(2,5-difluorophényl)-2-hydroxy-3-[4-(4-cyanophényl)thiazol-2-yl]butyl]-1H-[1,2,4]triazol-4-ium,
t) chlorhydrate de l'iodure de [[N-méthyl-N-2-(méthylamino)acétoxyméthyl-4,5-diméthoxy-phényl]carbamoyloxy]-méthyl-l-[(2R,3R)-2-(2,5-difluorophényl)-2-hydroxy-3-[4-(4-cyanophényl)thiazol-2-yl]butyl]-1H-[1,2,4]triazol-4-ium,
u) chlorhydrate du chlorure de 1-[[N-méthyl-N-2-(méthylamino)acétoxyméthyl-5-fluorophényl]carbamoyloxy]éthyl-1-[(2R,3R)-2-(2,5-difluorophényl)-2-hydroxy-3-[4-(4-cyanophényl)thiazol-2-yl]butyl]-1H-[1,2,4]triazol-4-ium,
v) chlorhydrate de l'iodure de 1-[[N-méthyl-N-2-(méthylamino)acétoxyméthyl-6-méthylphényl]carbamoyloxy]éthyl-1-((2R,3R)-2-(2,5-difluorophényl)-2-hydroxy-3-[4-(4-cyanophényl)thiazol-2-yl]butyl]-1H-[1,2,4]triazol-4-ium,
w) chlorhydrate de l'iodure de 1-[[N-méthyl-N-2-(méthylamino)acétoxyméthyl-4-chlorophényl]carbamoyloxy]éthyl-1-[(2R,3R)-2-(2,5-difluorophényl)-2-hydroxy-3-[4-(4-cyanophényl)thiazol-2-yl]butyl]-1H-[1,2,4]triazol-4-ium,
x) chlorhydrate du chlorure de 1-[[N-(méthylamino)acétoxyéthyl-N-2,4-difluorophényl]carbamoyloxy]éthyl-1-[(2R,3R)-2-(2,5-difluorophényl)-2-hydroxy-3-[4-(4-cyanophényl)thiazol-2-yl]butyl]-1H-[1,2,4]triazol-4-ium,
y) chlorhydrate de l'iodure de 1-[[N-méthyl-N-2-(méthylamino)acétoxyméthyl-5-chlorophényl]carbamoyloxy]éthyl-1-[(2R,3R)-2-(2,5-difluorophényl)-2-hydroxy-3-[4-(4-cyanophényl)thiazol-2yl]butyl]-1H-[1,2,4]triazol-4-ium,
z) chlorhydrate du chlorure de 1-[[N-méthyl-N-2-(méthylamino)acétoxyméthyl-5-nitro-phényl]carbamoyloxy]éthyl-1-[(2R,3R)-2-(2,5-difluorophényl)-2-hydroxy-3-[4-(4-cyanophényl)thiazol-2-y]]butyl]-1H-[1,2,4]triazol-4-ium,
bb) chlorhydrate du chlorure de 1-[[N-méthyl-N-2-(méthylamino)acétoxyméthyl-3-fluorophényl]carbamoyloxy]éthyl-1-[(2R,3R)-2-(2,5-difluorophényl)-2-hydroxy-3-[4-(4-cyanophényl)thiazol-2-yl]butyl]-1H-[1,2,4]triazol-4-ium,
cc) chlorhydrate du chlorure de 1-[[N-méthyl-N-2-(méthylamino)acétoxyméthyl-5-cyanophényl]carbamoyloxy]éthyl-1-[(2R,3R)-2-(2,5-difluorophényl)-2-hydroxy-3-[4-(4-cyanophényl)thiazol-2-yl]butyl]-1H-[1,2,4]triazol-4-ium,
dd) chlorhydrate du chlorure de 1-[[N-méthyl-N-2-(méthylamino)acétoxyméthyl-3-chlorophényl]carbamoyloxy]éthyl-1-[(2R,3R)-2-(2,5-difluorophényl)-2-hydroxy-3-[4-(4-cyanophényl)thiazol-2-yl]butyl]-1H-[1,2,4]triazol-4-ium,
ee) chlorhydrate du chlorure de 1-[[N-méthyl-N-2-(méthylamino)acétoxyméthyl-4-cyanophényl]carbamoyloxy]éthyl-1-[(2R,3R)-2-(2,5-difluorophényl)-2-hydroxy-3-[4-(4-cyanophényl)thiazol-2-yl]butyl]-1H-[1,2,4]triazol-4-ium,
ff) chlorhydrate de l'iodure de 1-[[N-méthyl-N-2-(méthylamino)acétoxyméthyl-5-trifluorométhylphényl]carbamoyloxy]éthyl-1-[(2R,3R)-2-(2,5-difluorophényl)-2-hydroxy-3-[4-(4-cyanophényl)thiazol-2-yl]butyl]-1H-[1,2,4]triazol-4-ium,
gg) chlorhydrate du chlorure de 1-[[N-méthyl-N-2-(amino)acétoxyméthyl-3-chlorophényl]carbamoyloxy]éthyl-1-[(2R,3R)-2-(2,5-difluorophényl)-2-hydroxy-3-[4-(4-cyanophényl)thiazol-2-yl]butyl]-1H-[1,2,4]triazol-4-ium,
hh) chlorhydrate du chlorure de 1-[[N-éthyl-N-2-(méthylamino)acétoxyméthyl-3-chlorophényl]carbamoyloxy]éthyl-1-[(2R,3R)-2-(2,5-difluorophényl)-2-hydroxy-3-[4-(4-cyanophényl)thiazol-2-yl]butyl]-1H-[1,2,4]triazol-4-ium,
ii) chlorhydrate du chlorure de 1-[[N-méthyl-N-3-[(amino)acétoxyméthyl]pyridin-2-yl]carbamoyloxy]éthyl-1-[(2R,3R)-2-(2,5-difluorophényl)-2-hydroxy-3-[4-(4-cyanophényl)thiazol-2-yl]butyl]-1H-[1,2,4]triazol-4-ium,
jj) chlorhydrate de l'iodure de 1-[[N-méthyl-N-2-(méthylamino)acétoxyméthyl-3-méthylphényl]carbamoyloxy]éthyl-l-[(2R,3R)-2-(2,5-difluorophényl)-2-hydroxy-3-[4-(4-cyanophényl)thiazol-2-yl]butyl]-1H-[1,2,4]triazol-4-ium,
kk) chlorhydrate du chlorure de 1-[[N-éthoxycarbonyl-N-2-(méthylamino)acétoxyméthylphényl]carbamoyloxy]éthyl-1-[(2R,3R)-2-(2,5-difluorophényl)-2-hydroxy-3-[4-(4-cyanophényl)thiazol-2-yl]butyl]-1H-[1,2,4]triazol-4-ium,
ll) chlorhydrate du chlorure de 1-[[N-pivaloyl-N-2-(méthylamino)acétoxyméthylphényl]carbamoyloxy]éthyl-1-[(2R,3R)-2-(2,5-difluorophényl)-2-hydroxy-3-[4-(4-cyanophényl)thiazol-2-yl]butyl]-1H-[1,2,4]triazol-4-ium.

17. Composé selon l'une quelconque des revendications 1 à 16 qui est le chlorhydrate du chlorure 1-[[N-méthyl-N-3-[(méthylamino)acétoxyméthyl]pyridin-2-yl]carbamoyloxy]éthyl-l-[(2R,3R)-2-(2,5-difluorophényl)-2-hydroxy-3-[4-(4-cyanophényl)thiazol-2-yl]butyl]-1H-[1,2,4]triazol-4-ium.

18. Composé de formule (V) qui est 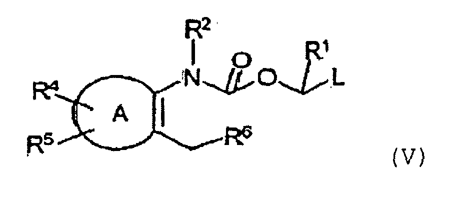 dans laquelle R¹, R², R⁴, R⁵, R⁶ et le groupe 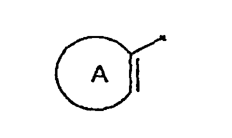 sont tels que définis dans l'une quelconque des revendications 1 à 17 et L est un groupe partant.

19. Processus pour la fabrication d'un composé de formule générale (III) tel que défini dans la revendication 1, qui comprend la réaction d'un composé azole possédant une activité antifongique de formule générale (II). 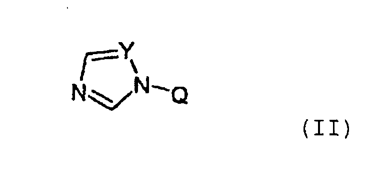 dans laquelle Y et Q sont tels que définis dans la revendication 1, avec un composé de formule générale (V) tel que défini dans la revendication 18.

20. Composés selon l'une quelconque des revendications 1 à 17, tel qu'obtenus par un processus selon la revendication 19.

21. Composition pharmaceutique, en particulier pour l'utilisation en tant qu'antifongique, comprenant un composé tel que défini dans l'une quelconque des revendications 1 à 17 et un transporteur pharmaceutiquement acceptable.

22. Composés tels que définis dans l'une quelconque des revendications 1 à 17, pour l'utilisation en tant que substances actives thérapeutiques, en particulier en tant que substances actives d'un point de vue antifongique.

23. Utilisation d'un composé tel que défini dans l'une quelconque des revendications 1 à 17, pour la préparation d'un médicament pour la prophylaxie et/ou le traitement des infections fongiques.
